# EUROPEAN PATENT APPLICATION

(11) **EP 2 674 162 A1**
(43) Date of publication of application: **18.12.2013**
(21) Application number: 13460042.8
(22) Date of filing: 29.05.2013
(51) Int. Cl.: A61K 35/74, A23L 1/30, A61L 29/08, A61L 29/16, A61P 19/06, A61P 31/04, A61P 1/00, C12R 1/225

(54) **NANOPRODUCT COMPRISING LACTOBACILLUS REUTERI DAN080 USEFUL IN PROPHYLAXIS AND MEDICINE, BOTH HUMAN AND VETERINARY AND MEDICAL USE OF THE SAME**

(30) Priority: 29.05.2012 PL 3912
(71) Applicant: Kruszewska, Danuta, 02-765 Warszawa (PL)
(72) Inventor: Kruszewska, Danuta, 02-765 Warszawa (PL)
(74) Representative: Malewska, Ewa

(57) **Abstract**

The invention relates to a use of *Lactobacillus reuteri* DAN080 strain with deposit number DSM 15693 as an active component for manufacturing a dermaceutic effective in skin infections in vertebrate, in particular human being, other mammal or bird, and for manufacturing a medical device in the form of a dressing or hygienic materials for use in a wound care and personal hygiene - respectively, to avoid skin infections in vertebrate, in particular human being, other mammal or bird, as well as for manufacturing a medical device in the form of plastic protector sheet and fire-blankets intended for rescue units, including fire-brigades, specifically for patients with extensive skin bums and for victims of communication accidents with serious body surface injuries, to avoid skin infections in vertebrate, in particular human being, other mammal or bird.

The *Lactobacillus reuteri* DAN080 strain with deposit number DSM 15693 is used in form of the *L. reuteri* DAN080 strain culture, partially inactivated *L. reuteri* DAN080 strain culture; liquid supernatant of *L. reuteri* DAN080 strain culture, concentrated supernatant of *L. reuteri* DAN080 strain culture and dried supernatant of *L. reuteri* DAN080 strain culture for prophylaxis and treatment of medical conditions developing as a result of infections caused by bacteria, fungi, and other pathogens of the body surface in vertebrate, and alternatively in form selected from the group comprising the whole *L*. *reuteri* DAN080 strain culture; liquid, concentrated and dried supernatants obtained from *L. reuteri* DAN080 strain cultures and from mixed bacterial cultures containing *L. reuteri* DAN080 strain and liquid, concentrated and dried supernatants obtained from the cultures of prokaryotic and eukaryotic recombinants, and the whole cultures of prokaryotic and eukaryotic recombinants, in which and/or from which genes are utilized, which genes provide specific modulatory, inhibitory, and homeostatic activity with respect to *H. pylori* and other bacteria; proteins/oligopeptides/peptides of molecular weight of approximately: 150 and/or 141 and/or 115 and/or 95 and/or 90 and/or 86 and/or 83 and/or 77 and/or 71 and/or 63 and/or 59 and/or 56 and/or 49 and/or 46 and/or 43 and/or 39 and/or 34 and/or 32 and/or 30 and/or 22 kD and lower, purified/isolated from liquid/concentrated/dried supernatant obtained from *L. reuteri* DAN080 strain [cultures] and purified/isolated from the whole *L. reuteri* DAN080 strain cultures and from other mixed bacterial cultures, which include *L. reuteri* DAN080 strain and from purified or isolated cultures of prokaryotic and eukaryotic recombinants, in which and/or from which genes are utilized, which genes provide specific modulatory, inhibitory, homeostatic activity with respect to *Helicobacter pylori* and other bacteria or their mixtures for prophylaxis and treatment of medical conditions developing as a result of infections caused by bacteria, fungi, and other pathogens of the alimentary tract, body surface and other systems, such as urogenital, respiratory systems in vertebrates.

The present invention provides for a protection against medical conditions of body surface in vertebrates, including mammals and birds and for treatment of those conditions.

## Description

The invention relates to a dermaceutic and new medical devices comprising the same, as well as to the methods for manufacturing them and use. The source of an active substance for the dermaceutic and for the new medical devices comprising the same is a new microorganism isolated and identified by the present inventor - Danuta Kruszewska.

In an organism of a healthy human (and also of various animals), including body surface, microorganisms are present throughout the lifetime of the organism. Among these microorganisms, bacteria prevail, although fungi and protozoa are also present. Various types of microorganisms colonize most intensively the mucosa of the gastrointestinal tract, body surface and other systems, e.g. urogenital or upper airways, and settle on mucosal surfaces. The mechanisms, according to which individual species/strains of a specific microbiota, as well as the microbiota as a whole perform their function in the maintenance of health, have not been fully recognized. Microbiota of the gastrointestinal tract, the skin, and other ecological niches inhabited by microbes differ with respect to the functions performed, composition and amount of microorganisms. Thus, the term 'microbiota' is to be understood as referring to a 'herd' of microorganisms inhabiting in a specific anatomical and physiological region of the body, which constitutes a microbiom of the microorganisms.

Conditions prevailing within the well explored microbioms such as human gastrointestinal tract provide a good illustration of the present invention as various inter-relations and mechanisms observed here can also be found in other microbioms of living organisms.

Mutual interactions of microorganisms living in various microbioms have the character of a symbiotic co-existence. Microbiota of individual microbiom stimulate host immune system, not only on the level of a microorganism (live or dead), but also through its intra- and extra-cellular metabolites, counteract the anchoring of other microorganisms dangerous for health (mainly pathogens) - through competitiveness with respect to receptor and substrate, neutralize toxic intra- and extra-cellular metabolites of other microbes, regulate the development and physiological functions of the specific microbiom. The complex participation of the microbiota or disfunction of the same in development of various diseases, have thus far been rarely taken into consideration.

For example, *Helicobacter pylori* is a microorganism colonizing gastric and duodenal mucous membranes, responsible for a number of medical conditions, including gastritis and other related diseases, such as gastric and duodenal ulcer, stomach (gastric) cancer, duodenal cancer, intestinal disorders, disorders of the gastrointestinal tract (GIT) and diarrhoea which, in fact, afflict every representative of the human species living in the modem societies of Western countries. This is the main medical problem for 1/3 of the human population, which has growing medical, social and economic consequences.

In the alimentary system there also occur beneficial lactic acid bacteria (LAB) which, as such, as well as products of their metabolism, are considered a part of natural health ameliorating microbiota and as agents improving the health of the host and the intestine (see: Kullisaar T, Zilmer M, Mikelsaar M, Vihalemm T, Annuk H, Kairane C, et al. Two antioxidative lactobacili strains as promising probioticts. Int J Food Microbiol 2002; 72: 215-24).

LAB are generally recognized as safe (GRAS) bacteria. Despite the outstanding effect on the general health status, especially the effect on the GIT, LAB bacteria show an insufficient capability of GIT colonization after the period of breast suckling in mammals, and generally, for the whole life-time in birds. This is due, firstly, to the deficiency of suitable substrates for the growth of LAB after the period of breast suckling, and secondly to the characteristics of LAB bacterial wall which is incapable of producing fimbriae, etc., responsible for the bacterial adhesion to the GIT epithelium or to the GIT mucosa.

Many LAB are currently available in commerce, offered, as live cultures, food additives for humans and feed additives for animals, such as for example, *Lactobacillus plantarum* 299 v and others. It is expected that the metabolites of LAB growing in the alimentary system will be beneficial for the consumer of these bacteria. This is very often controversial, because live LAB cultures are excellently eliminated and killed by antibacterial compounds of the host, which basically reduce the growth of bacteria in stomach and the upper part of intestine.

Lysozyme - a hydrolytic enzyme released by certain phagocytes, such as macrophages and multinuclear leukocytes, plays a significant role in the control of pathogenic microorganisms. Lysozyme is also produced by Paneth cells located in the lining of the intestines. Lysozyme is especially active against Gram-positive microorganisms. Phagocytic activity of cells involves degradation, with the participation of lysozyme, of the cellular wall of bacteria, more precisely - a cleavage of glycoside bonds in peptidoglycans.

Despite the possibilities of isolation and full identification of lysozyme, the therapeutic use of this compound is not possible due to its high activity. In the publication No. WO 89/11294 a possibility of the therapeutic use of the dimerized form of lysozyme is disclosed indicating its effectiveness in therapeutic use in human and veterinary medicine.

In medical science, and also in animal husbandry (zootechny) and veterinary medicine frequently development of undesired phenomena and medical conditions is observed as a result of an attack or assault of microorganisms on a specific anatomic or physiologic areas of the body, including body surface damaged by wounds and bums, as well as on instruments, medical equipment and even prostheses, the microorganisms being able to colonize the same and to participate in pathogenesis of the undesired phenomena and medical conditions.

Representative example of medical devices responsible for exposing a patient using the same to such a risk are ureologic catheters. Apart from undoubted benefits, undesirable effects are also associated with the use of catheters and other medical equipment remaining in contact with patient's body tissues. Infections related to the use of catheters are usually caused by bacteria responsible for formation ofbiofilm on the catheter surface.

Biofilm is generated by various microbes, including ureolytic bacteria, non-pathogenic commensals, permanently and naturally colonizing the surface of the epithelium, pathogens - including microorganisms causing urogenital system infections (e.g. *Proteus mirabilis*). The common feature of ureolytic bacteria is their ability to make use of urea present in the environment (tissues) - mainly as a source of nitrogen necessary for survival, which use involves urease. Urease (including bacterial urease), hydrolyses urea to ammonia and carbon dioxide. Examples of nitrogen-assimilating bacteria by means of their own ureases are biofilm forming bacteria.

In the prior art, attempts have been undertaken to enhance the usability values of catheters to be applied in human and veterinary medicine, by coating them with chemotherapeutics and impregnation with antiseptics or other agents (e.g. anticoagulants). The first remedy is the application of hydrogel on the catheter surface directly before catheterization in order to decrease its friction coefficient and to reduce pain. Hydrogel is mainly composed of polyvinyl pyrrolidone (***PVP***), usually in combination with iodide acting antiseptically. However, it requires additional operations and the gel applied is easily rinsed away, therefore the antiseptic action is limited in time. More advanced coatings are permanently attached to the catheter surface and allow decrease of the friction coefficient, not only during insertion, but also during catheter removal. A permanent hydrogel coating may also serve as an antibacterial drug reservoir that slows down colonization of the surface. Nevertheless, the problem of infections associated with long lasting catheterization has not been fully resolved. In the case of long term placement of a catheter within the organism, the process of antibacterial agent release should be controlled and slow to maintain its bactericidal properties for the whole period of catheterization, which has not been yet accomplished in a sufficient degree.

Similar phenomena take place on the surface of other medical devices remaining in contact with the patient's body tissues.

There are many known methods of application of antibacterial coatings on natural or polymer tubes. The hydrogel coating technique is advantageous due to high biocompatibility of the coating, low friction, decreased bacteria adhesion and a possibility of incorporation of drug into the coating.

In EP 1 917 959 the use of alpha-ketoglutarate for production of an agent against the deposits and infectious stones formation in the urogenital system by ureolytic bacteria has been disclosed.

It is thus the main aim of the present invention to provide a new dermaceutic and medical devices for body surface pathogens elimination or growth control, in vertebrates including humans, other mammals and birds, that use such medical devices.

Another aim of the invention is to provide a new antibacterial and antimicrobial agent as dermaceutic for prophylaxis and treatment of medical conditions developing as a result of infections induced by bacteria, fungi and other pathogens of the body surface in vertebrates, including humans, other mammals and birds.

Also, it is the aim of the invention to provide medical devices such as dressings and hygienic materials for use in personal hygiene, comprising a natural antibacterial and antimicrobial agent, effective in prophylaxis and treatment of medical conditions developing as a result of infections induced by bacteria, fungi and other pathogens of the body surface, as well as plastic protector sheets and fire-blankets - for rescue units, including fire-brigades, intended for patients with extensive skin burns and for victims of accidents (e.g. road traffic accidents) with serious body surface injuries.

Further important aim of the invention is making use of nanotechnology to provide antimicrobials ensuring endogenous biodegradation for body fluids and tissues, acting from the surface of the above mentioned medical devices such as dressings and hygienic materials as well as plastic protector sheets and fire-blankets for rescue units.

The above-mentioned and other goals were unexpectedly achieved due to the development of a technical solution according to the present invention, as claimed in the appended claims, based on the isolation and identification of a new microorganism - *Lactobacillus reuteri* DAN080, deposited on June 20, 2003 in DSMZ collection (Deutsche Sammlung von Mikroorganismen und Zellkulturen GmbH, in Braunschweig, DE), access number: DSM 15693 - in accordance with a Budapest Treaty on the International Recognition of the Deposit of Microorganisms for the Purposes of Patent Procedure.

The invention relates to a new dermaceutic and medical devices comprising the same, that is comprising cultures of *L. reuteri* DAN080, the partially inactivated cultures of *L. reuteri* DAN080, liquid supernatants of *L. reuteri* DAN080 cultures, concentrated supernatants of *L. reuteri* DAN080 cultures, and dried supernatants of *L. reuteri* DAN080 cultures, as the active component, in particular antimicrobial component for prophylaxis and treatment of medical conditions developing as a result of infections induced by bacteria, fungi and other pathogens of the body surface [in vertebrates].

The active component of the dermaceutic according to the present invention is selected from a group comprising the whole culture of *L. reuteri* DAN080, liquid supernatant, concentrated supernatant, and dried supernatant obtained from the culture of *L. reuteri* DAN080, and from mixed bacterial cultures comprising *L. reuteri* DAN080, and liquid supernatants, concentrated supernatants and dried supernatants obtained from cultures of prokaryotic and eukaryotic recombinants and whole cultures of prokaryotic and eukaryotic recombinants, in which and/or from which genes are utilized, which genes provide specific modulating, inhibitory, homeostatic activity against *H. pylori* and other bacteria, as well as proteins/oligopeptides/peptides of the molecular weight of approximately: 150 and/or 141 and/or 115 and/or 95 and/or 90 and/or 86 and/or 83 and/or 77 and/or 71 and/or 63 and/or 59 and/or 56 and/or 49 and/or 46 and/or 43 and/or 39 and/or 34 and/or 32 and/or 30 and/or 22 kD and lower, purified/isolated from liquid/condensed/dried supernatant obtained from *L. reuteri* DAN080 [cultures] and purified/isolated from whole cultures of *L. reuteri* DAN080, and from other mixed bacterial cultures comprising *L. reuteri* DAN080, and purified or isolated from cultures of prokaryotic and eukaryotic recombinants, in which and/or from which genes are utilized, which genes provide specific modulating, inhibitory, homeostatic activity against *H. pylori* and other bacteria, or mixtures thereof for prophylaxis and treatment of medical conditions developing as a result of infections induced by bacteria, fungi and other pathogens of the body surface in vertebrates, or else for treatment and prevention of the development of the above mentioned medical conditions and/or for increasing the activity of lysozyme in the organism of vertebrate, especially human, other mammal and bird.

According to the invention the vertebrate is human individual.

The vertebrate is also a domestic animal, pet, animal involved in sport, broiler, layer hen, mouse, rat, guinea pig, rabbit and other laboratory animal, including primates, independently of its age.

According to the invention the microorganism is a pathogenic bacterium or fungi.

The invention relates also the use of the active component for manufacturing of a dermaceutic for modulation of functioning of body surface and/or for increasing the activity of lysozyme in the organism of vertebrate in need of such a treatment, including human being, other mammal or bird, which dermaceutic comprises the active component in an effective amount, that provides for obtaining the desired prophylactic or therapeutic effect.

According to the invention the dermaceutic is intended to kill, inhibit, control, and prevent the growth of microorganisms on the body surface and/or to increase the activity of lysozyme in the organism of vertebrate, especially human being, other mammal and bird, and to be administered in an effective amount and at a sufficient rate, necessary for reaching the desired prophylactic or therapeutic result.

In particular, the dermaceutic is a pharmaceutical composition comprising optionally, other biologically active substances, such as vitamins, especially vitamins D and E, especially in a nanoform, salts of lactic acid and other acids comprised in the *L*. *reuteri* DAN080 metabolites, in preventive or therapeutic doses, and pharmaceutically acceptable carriers and/or adjuvants.

Preferably, the dermaceutic according to the invention for increasing the activity of lysozyme in the organism of vertebrate, is introduced to the patient organism though the tissues of body surface or through systemic routes of administration for example orally if form of tablets or capsules - when in a solid form and divided into single doses comprising therapeutically effective amount of the above mentioned active component, preferably in the amount of from 0.001 to 0.2 g/kg of body weight per day.

The dermaceutic of the invention may be in a liquid or semisolid (ointment, cream or gel) form comprising a therapeutically or preventively effective amount of the above identified active component, preferably in the amount of from 0.001 to 0.2 g/kg of body weight per day, when applied on a body surface - skin, once or several times a day, suitable for administration in prophylaxis or treatment of body surface infections. The dermaceutic - when in a liquid form, may be topically used as aerosol, cataplasm or moist compress, or via systemic administration route as injection or in form of a drip.

Especially preferably in accordance with the invention, in the dermaceutic or in medical device as the active component there is used the product of fermentation of *Lactobacillus reuteri* DAN080 is used in form of cultures, at least partially inactivated cultures and the supernatants of these cultures only - respectively processed, for the modulation of the function of body surface and/or for increasing the activity of lysozyme in the organism of vertebrates in need for such treatment, including humans, other mammals and birds.

Hereinafter demonstrated biological activity of the live cultures of the new bacterial strain *L. reuteri* DAN080 and of the above mentioned form derived therefrom, allows those skilled in the art to apply the present invention in prophylaxis and treatment of medical conditions developing under influence of pathogens able to colonize the patient body surface, wherein the patient is human being, as well as other mammals and birds, and to determine appropriate administration routes.

As in another aspect of the present invention, the composition comprising the above active component is intended to stimulate the immune system via increasing the activity of lysozyme in the organism of vertebrate to enhance action of dermaceutic or medical device of the present invention, one may consider a dietary supplement, food or beverage (for humans) or fodder (for animals) additive as a vehicle to provide the patient with the same active component, in the course of the treatment with the dermaceutic and/or using the medical device of the present invention.. The dietary supplement, food or beverage or else a fodder additive is in a solid form and/or in form of beverage. Preferable amount of the active component is from 0.001 to 0.2 g/kg of body weight per day. Such a dietary supplement, food or beverage, or else fodder additive optionally contains other biologically active substances and vitamins D and E, especially in the form of nanoparticles, in preventive doses.

The solution according to the invention allows the restoration of the normal skin (body surface) condition and has a stimulatory effect on the immune system, especially by increasing lysozyme activity and enhancing its antimicrobial and antiviral activity.

As it has been mentioned above, the present invention is directed to special medical devices such as dressings, hygienic materials as well as plastic protector sheets and fire-blankets for rescue units.

At present, it has been unexpectedly found out that the above-mentioned and other goals may be achieved by a solution based on coating the surface of the medical devices with nanocoatings of various substances, primarily including the active component originating from the above identified new strain of lactic acid bacteria - *L. reuteri* DAN080, in the composition of at least one of the nanocoatings, the component being also able to decrease stress and to induce the patient's defensive immune response, thus reducing the risk of viral and bacterial infections.

Surprisingly, it was also observed that incorporation of specific vitamins in nano-form and alpha-ketoglutarate into nanocoatings of the medical devices according to the present invention provides a synergistic effect with respect to the prevention of infections and inflammations of the tissues remaining in a contact with the devices.

The active component used in the dermaceutic of the invention, may be used in form of dressings and hygienic materials for personal hygiene, if at least the layer being in contact with patient's tissues us saturated with the active component.

It is particularly preferred to use the active component to coat, preferably with a nanolayer, plastic protector sheets and fire-blankets for rescue units - including fire-brigades, intended for patients with extensive body surface injuries caused by road traffic accidents or by fires.

The medical devices such as above mentioned dressings or hygienic materials on one hand and plastic protector sheets and fire-blankets - on the other, comprise the active component, whereas the dressings, hygienic materials, etc. are impregnated with the active component, while the plastic protector sheets and also fire-blankets are coated with a nanolayer comprising the active component.

In accordance with the invention, the outer nanocoating for contact with patient's body tissues is made of biocompatible polymer, optionally capable of forming gel with water, permanently attached to said plastic either directly or through a nanocoating of polymer chemically bonded to the basic material and having antibacterial properties, wherein at least one of the nanocoatings comprises an addition of the above indicated active component, that is the cultures and/or extracellular and intracellular metabolites secreted by *Lactobacillus reuteri* DAN080, said metabolites having antimicrobial and anti-inflammatory activity, as well as an optional addition of vitamin D and E in form of nanoparticles.

According to the invention the biocompatible polymer is polyvinyl pyrrolidone and thickness of the nanocoating made of this polymer is about 50,000 C-C bonds (10 nm).

Preferably, the polymer having antibacterial properties is a salt of chitosan and small organic acid, preferably alpha-ketoglutaric acid.

According to the invention, in nanocoating of biocompatible polymer and/or in nanocoating of polymer having antibacterial properties there are dispersed additional active agents selected from the group comprising chitosan alpha-ketoglutarate, chitosan citrate, chitosan lactate with antimicrobial and anti-inflammatory activity, small dicarboxylic acid, silver nanoparticles, vitamins D and E - preferably in the form of nanopowder coated with a protective coating, and combinations thereof.

AS it is shown below, the above identified active component also has a stress-reducing properties. Based on those properties, the present invention also covers a rescue kit comprising the medical device and a stress-reducer for administration *per os,* in form of live or thermally inactivated cultures of *Lactobacillus reuteri* DAN080 at a dose of 10⁶ cells, for administration for the period of using the medical device.

In accordance with the invention nanocoatings has been developed for use on medical devices of the invention, in particular made of plastics. The nanolayers not only decrease the friction coefficient, thus reducing pain experienced by the patient, but also contain an additive being an agent which in contact with the patient's body tissues reduces patient's stress associated with use of the devices. Simultaneously, the same additive induces an increase of lysozyme level in tissues remaining in contact with the medical device, thus decreasing the risk of viral, bacterial infection, due to a very wide spectrum of lysozyme activity. The new nanocoating on the surface of the medical device contains also other active substances released gradually in a controlled manner.

Surface nanoengineering applied in accordance with the present invention allows for providing a coating which releases a medicament on demand. One of the signals triggering release of the drug may be, for example, a change of pH of the environment caused by bacterial growth. This targeted drug release is much more effective and shows less side effects when compared to traditional products releasing therapeutic components spontaneously or employing a prolonged release system. In addition, by the proper selection of the composition of coating layers dedicated medical devices are obtained, having varied destination, adjusted to the patient's status. The use of a coating made of appropriate coating with a chemically bonded drug, such as for example small dicarboxylic acid, ensures achievement of the goals assumed.

The present medical device (made of plastic) of the invention ensure the expected progress in medical care due to the possibility of making use of nanotechnology for delivery of natural antimicrobial agents, acting on the surface of the medical device according to the invention. The medical device being coated with nanocoating is safer for a patient. The coating reduces pain connected with contact with a patient's tissue and significantly decreases the probability of infection. Moreover, all the active substances used do not induce any undesirable side effects in the patient.

Due to the present invention based on the use of nanotechnology, the role of protective coatings on the medical devices of the invention is maximized thanks to enhancement of antimicrobial properties of nanocoatings and targeted release on demand of an active substance. It is most important that the medical devices according to the invention will save pain for patients and decrease the number of infections, while in view of character of the compounds employed eliminate the development of drug-resistance in microorganisms induced by classic antibiotics.

In accordance to the invention, the new dermaceutic can be used in superficial (topical) applications on skin - the body surface, for the remarkable antimicrobial activity of the above identified active component of the dermaceutic, also in combination with various absorbents including liquid absorbent materials. Non-limiting examples of corresponding medical devices include diapers, tampons, bandages, bandaids, sanitary pads, sanitary napkins with wings, panty liners, cosmetic pads, wraps for animals for night and day use. This group of articles can be made of fibers, with ultrathin (silk-thin and soft) cotton surface layers. The present active component incorporated in diapers can help in the prevention of hip rash and tender skin care. Such diapers in various sizes, with adjustable buckle can be used by children and adults.

The fire-blankets can be manufactured according to the new European standards for fire-blankets. They are designed with extra flexibility, using specially selected materials coated or containing the present active component used in accordance with the present invention, having therapeutic and prophylactic activity and may be better adjusted to smother flaming objects or clothing. They can be fully asbestos free and will not fray. The fire-blankets may be packed into quick release flexible wallets but can also be packed into containers, depending on the final users' requirements. They can take different size up to 180 cm². It is foreseen that each square centimeter of their surface can release in use an effective amount of the above mentioned active component.

The benefit of this particular aspect of the present invention is such that when a potentially tragic in-door fire disaster takes place, it provides an immediate therapeutic treatment to bum victims that might require primary reconstructive bum surgery. Bum patients often have bums of differing sizes (smaller vs larger percentage of total body surface area) and degrees of severity, with some requiring escharotomies, fasciotomies, primary excisions, skin grafts, amputations, local flaps, free flap coverage, thoracic surgery, etc. with long periods of staying in hospital, of more than 150 days. Severe infections can also occur if not immediately prevented.

In the present application exemplary pathogenic bacterium is *H. pylori.* Although some tests carried out with the active component used according to the present invention refer to the studies employing *H. pylori,* the observation and conclusions may be used to illustrate the present invention.

### Brief description of drawings

Further goals and advantages resulting from the present invention will be discussed in details below in the detailed description of the invention, with the reference to the drawings, where:
Fig. 1 - presents in graphic form the results of plate diffusion assay (agar medium GAB-CAMP with the strain *H. pylori* 17874): 1 - non-active supernatant obtained from the culture of *L. reuteri*; 2 - non-active broth MRS; 3 and 4 - inhibition zones caused by the activity of the active supernatant obtained from the culture of *L. reuteri* DAN080;
Fig. 2 - illustrates the effect of supernatant from the culture of *L. reuteri* DAN080 on the growth of *H. pylori* in a liquid medium - BHI broth;
Fig. 3 - presents SDS-PAGE electrophoresis of supernatants from *L. reuteri* DAN080 obtained, respectively, after 1, 2, 3, 4, 5, 6, 8 and 10 hours of growth in MRS broth. Numbers 1 - 20 designate the identified proteins released into the medium by the *L*. *reuteri*;
Fig. 4 - illustrates the identification of *L. reuteri* DAN080 by Denaturating Gradient Gel Electrophoresis *L. reuteri* DAN080 PCR product amplified with primers LacF and LacR;
Fig. 5 - shows the relationship between the activity of lysozyme (U/L) in rat blood, and the presence of the bacteria *L. reuteri* DAN080 in their gastrointestinal tracts, following intragastric administration of *L. reuteri* DAN080 - 10⁶ cells *Lactobacillus reuteri* DAN080; DAN080P - thermally killed 10⁶ cells *Lactobacillus reuteri* DAN080; ChAKG - chitosan alpha-ketoglutarate; SF - saline;
Fig. 6 - presents the relationship between the activity of neurons isolated from the Enteric Nervous System and extracellular metabolites of *L. reuteri* DAN080;
Fig. 7 - illustrates the activity of lysozyme (U/L) in rat blood following intragastric administration of *Lactobacillus reuteri* DAN080.

### Detailed description of the invention

Bacteria *L. reuteri* DAN080 were isolated from the gastrointestinal tract of a healthy laboratory animal. The bacteria were isolated as a single colony on a solid medium with blood agar. This medium was incubated with the scrapings from the gastrointestinal tract of a healthy mouse at the temperature of 37°C for 24 hours. The.colony isolated was proliferated in the broth MRSB (Oxoid), on a standard medium for lactic acid bacteria (LAB). The pH of the medium prior to sterilization was pH 6.8. Sterilization was performed within 15 min., at temp. of 121°C, the pH of the medium after sterilization: pH 6.2. The thermal conditions of bacteria incubation remained within the range 35±3°C. The full growth in the liquid culture was 16 hours. The bacteria can be stored, with warranty of survival at the temperature of -20°C. *L. reuteri* DAN080, survive at room temperature +20 to +22°C for at least 30 days maintaining the capability of inhibiting the growth of other microorganisms.

In order to obtain an enhanced antimicrobial activity the bacteria *L. reuteri* DAN080 are grown on media comprising AKG.

Composition of the medium: 0.5% beef meat extract; 0.5% yeast extract; 1% peptone; 0.3 % NH₄Cl; 0.4 % K₂HPO₄; 0.4 % KH₂PO₄; 0.01% MgSO₄x7 H₂O; 0.005 % MnSO₄x4 H₂O; 0.1% Tween 80; 0,05 L-cysteine HCl; 0.0002 of each of the following vitamins: B1, B2, B6, B5, B12, B9. After sterilization in the conditions as presented above, 23 mmol/l maltose and, respectively, starch or glucose, and 10 mmol/l AKG were added to the medium. The medium pH was: pH 6.2. The bacteria were incubated at 37°C for 16 hours. An enhanced bacterial growth occurred in the presence of AKG. In the medium, from 4 to 6 mmol/l of acetates and lactates were found, while in the medium non-enriched with AKG - from 1 to 2 mmol/l.

*Lactobacillus reuteri* DAN080 was identified in accordance with biochemical activity, where the capability of fermenting carbohydrates was assessed by the test - Api 50 CH and CHL medium, bioMerieux SA, Marcy 1Étoile, France.
Taxon: *L. reuteri*
- phenotypic characteristics according to the API system
   CAT: 1053 1121 0000 000 000 0000000
   API RID32s: 515 151 511 111 315 111 111 511 111 111 11 1
   API50CHL: 1111533111 5511111111 1411111155 5511151111 1111111311
   Api ID32AN: 155 515 111 114 111 513 351 351111 312

Genotypic characteristics [SEQ. ID NO: 1, 2, 3]: based on DNA analysis and comparison with 16S gene sequence of rhibosomal RNA *L. reuteri* (see: GenBank: EF187261.2; Byun R, Nadkarni MA, Chhour KL, Martin FE, Jacques NA, Hunter N. Quantitative analysis of diverse Lactobacillus species present in advanced dental caries. J Clin Microbiol. 2004;42(7):3128-36; Fredricks DN, Relman DA. Improved amplification of microbial DNA from blood cultures by removal of the PCR inhibitor sodium polyanetholesulfonate. J Clin Microbiol. 1998; 36(10):2810-6). The following primers were useful for sequencing [SEQ. ID NO: 2, 3] - primer 1: TGGAAACAGA TGCTAATACC GC (22 bp) [SEQ. ID NO: 2], primer 2: ATTAGATACC CTGGTAGTCC (20 bp) [SEQ. ID NO: 3]

tggaaacaga tgctaatacc gcataacaac aaaagccaca tggcttttgt
ttgaaagatg gctttagcta tcactctggg atggacctgc ggtgcattag
ctagttggta aggtaacggc ttaccaaggc gatgatgcat agccgagttg
agagactgat cggccacaat ggaactgaga cacggtccat actcctacgg
gaggcagcag tagggaatct tccacaatgg gcgcaagcct gatggagcaa
caccgcgtga gtgaagaagg gtttcggctc gtaaagctct gttgttggag
aagaacgtgc gtgagagtaa ctgttcacgc agtgacggta tccaaccaga
aagtcacggc taactacgtg ccagcagccg cggtaatacg taggtggcaa
gcgttatccg gatttattgg gcgtaaagcg agcgcaggcg gttgcttagg

tctgatgtga aagccttcgg cttaaccgaa gaagtgcatc ggaaaccggg
ccacttgagt gcagaagagg acagtggaac tccatgtgta gcggtggaat
gcgtagatat atggaagaac accagtggcg aaggcggctg tctggtctgc
aactgacgct gaggctcgaa agcatgggta gcgaacagga ttagataccc
tggtagtcc [SEQ. ID NO: 1] (659 bp)

After a specified time of *L. reuteri* DAN080 growth, the culture is centrifuged and the liquid supernatant, concentrated supernatant, and dried or lyophilized supernatant is the product of a specific capability and activity for regulating the growth of *H. pylori* and other bacteria *in vitro* and *in vivo.* After electrophoretic separation performed from the liquid supernatant, the concentrated supernatant and dried supernatant collected at a predetermined time, specific proteins of a molecular weight within the range 150 - 22 kD or less were visualized, which proteins are responsible for homeostasis and regulation of the growth of *H. pylori.* These bands are weak, and do not occur in electrophoregraphs obtained from liquid supernatant, concentrated supernatant and dried supernatant harvested at a time different than the above-specified time for *L. reuteri* DAN080 culture, and the liquid supernatant, concentrated supernatant and dried supernatant, show one of the proper effects - homeostatic and growth regulating, on *H. pylori* and other bacteria *in vitro,* as well as *in vivo.*

For the genetic identification of *L. reuteri,* the total genomic DNA was isolated from bacteria cultured overnight, with the use of the kits DNaesy™, Qiagen GmbH, Hilden, Germany.

Amplification of the 340 bp fragment of 16S rDNA was performed with semi-nested PCR (first run: 94°C for 30 s., 61°C for 60 s., 68°C for 60 s., 35 cycles; second run: 94°C for 30 s., 58°C for 60 s., 68°C for 60 s., 40 cycles) using primers [SEQ. ID NO: 4, 5, 6] (see: Walter, J., Hertel, Ch., Tannock GW., Lis CM., Munro K., Hammes W.P. (2001) Detection of Lactobacillus, Pediococcus, Leuconostoc i Weisella species in human faeces by using group specific PCR primers and denaturing gradient gel electrophoresis. Appl. Environ. Microb. 67, 2578-2585); forward primer 3: AGCAGTAGGG AATCTTCCA (19 bp) [SEQ. ID NO: 4]; reverse primer 4: ATTYCACCGC TACACATG (18 bp) [SEQ. ID NO: 5]; forward primer 5: ACAATGGACG AAAGTCTGAG TG (22 bp) [SEQ. ID NO: 6].

### Definitions

The terms used in the presented description should be understood in their common basic meaning, unless defined otherwise below.

As used herein, the term 'killing, inhibiting, regulating, preventing growth of *H*. *pylori* and other bacteria, is intended to mean the pharmacological, chemical, mechanical and physiological characteristics of *L. reuteri* DAN080 cultures, partially inactivated *L*. *reuteri* DAN080 cultures, liquid supernatants of *L. reuteri* DAN080 cultures, condensed supernatants of *L. reuteri* DAN080 cultures and dried supernatants of *L. reuteri* DAN080 cultures, as measured by means of certain parameters applied in accordance with the invention. Such parameters are known to those skilled in the art, and are further defined in the presented description.

According to the meaning defined in the present description and claims, the term 'dermaceutic composition' means therapeutically and/or preventively effective composition of the invention comprising *L. reuteri* DAN080 cultures, partially inactivated *L. reuteri* DAN080 cultures of, liquid supernatants of *L. reuteri* DAN080 cultures, concentrated supernatants of *L. reuteri* DAN080 culture and dried supernatants of *L. reuteri* DAN080 cultures.

The term 'therapeutically effective amount' or 'effective amount' or 'therapeutically effective' applied in the presented description and claims refers to the amount of active antimicrobial agent used in accordance with the invention, that provides the therapeutic and/or preventive result when used in a specific condition and a specific administration regime. The term means a predetermined amount of the active material calculated so as to produce the desired therapeutic and/or preventive effect. The above-mentioned active material may be combined with an appropriate additive, for example, other microorganisms or diluents or carrier or administration vehicle. In addition, the term is intended to mean an amount sufficient to reduce, and most preferably to prevent a clinically significant deficiency in vertebrates in need of such treatment, the vertebrates including mammals and birds. The determination of a therapeutically effective amount remains within the scope of skills of a person skilled in the art and depends on the activity of the product according to the invention, place of activity, and innate sensitivity of vertebrates in need of the treatment, such vertebrates including mammals and birds. Alternatively, a therapeutically effective amount is sufficient to cause an improvement in the host's clinically significant condition.

As used herein, 'treatment' means treatment in order to cure, which may be a complete or partial recovery from the condition, disorders or states, subjected to the treatment.

As used herein, the term 'alleviation' means the reduction, i.e. less severe or milder condition, disorders or states subjected to the treatment.

As used herein, the term 'prevention' or 'prophylaxis' means a complete or partial inhibition of the development or outbreak of the defined condition, disorder, state or states. Determination of a preventively effective amount remains within the scope of skills of a skilled artisan, and depends on the activity of the product, place of activity and innate sensitivity of individual vertebrates in need of such treatment, the vertebrates including a mammals and birds. Alternatively, a preventively effective amount is sufficient to protect the host against such conditions, disorders or states.

With respect to the other aspects of the present invention it is essential to properly understand the following terms:
"Nanocoating" means known nanogel (polyvinyl pyrrolidone - PVP or other), in which polymers used are chemically bonded to chitosan salts of antimicrobial activity (citric or lactic or alpha-ketoglutaric acid salts, or mixtures thereof in various proportions and amounts).

Chitosan salts of antimicrobial activity (citric or lactic or alpha-ketoglutaric acid salts, or mixtures thereof in various proportions and amounts) may also form a coating which may be coated with a known nanogel in a non-modified form or modified with the above-mentioned chitosan salts of antimicrobial activity.

The subsequent coating may form vitamin D alternatively with chitosan salts of antimicrobial activity (citric or lactic or alpha-ketoglutaric acid salts, or mixtures thereof in various proportions and amounts), and a final coating of hydrogel in non-modified form or modified with chitosan salts of antimicrobial activity (citric or lactic or alpha-ketoglutaric acid salts, or mixtures thereof in various proportions and amounts).

Also possible is separate stratification of the medical device of the present invention with extracellular metabolites of *L. reuteri* DAN080 as deposited under the number: DSM 15693 and with silver or to modify therewith the nanogel which is coating the medical device according to the present invention on a side facing the body surface tissues of the patient.

The claimed dermaceutic and medical devices according to the present invention provide a treatment, alleviation or prevention of the growth of bacteria in various medical conditions of body surface and comprise cultures of *L. reuteri* DAN080, partially inactivated cultures of *L. reuteri* DAN080, liquid supernatants of *L. reuteri* DAN080 cultures, concentrated supernatants of *L. reuteri* DAN080 cultures and dried supernatants of *L. reuteri* DAN080 cultures, when administered to vertebrates, including mammals and birds, in a sufficient amount and optionally, at a sufficient rate capable of inducing the desired effect.

The changes improving the health status and functioning of body surface of vertebrates subjected to the treatment are compared with the health status and functioning of body surface in vertebrates which are not recipients of the antimicrobial agent comprised in the dermaceutic and/or medical device of the invention. The changes are considered as an improvement if they are beneficial for the vertebrates in need of such treatment, including mammals and birds.

According to the present invention, *L. reuteri* DAN080 cultures, partially inactivated *L. reuteri* DAN080 cultures, liquid supernatants of *L. reuteri* DAN080 cultures, concentrated supernatants of *L. reuteri* DAN080 cultures and dried supernatants of *L. reuteri* DAN080 cultures are used as the therapeutic and prophylactic agent in the dermaceutic and medical devices of the invention.

The present the dermaceutic and/or medical devices of the invention are used in treatment of vertebrates including mammals and birds - for instance, humans, domestic animals, pets, animals involved in sports, broilers, layer hens, mice, rats, guinea pigs, rabbits and other laboratory animals, including primates, independently of their age.

The liquid supernatant, condensed supernatant and dried supernatant obtained from the *L. reuteri* DAN080 culture may be included in the dermaceutic and/or medical devices of the invention in a manner selected according to the type of vertebrate to be treated, the condition of the vertebrate in need of the treatment and the specific indication for treatment.

A topical use of the dermaceutic and/or medical devices of the invention may be associated with one embodiment with systemic administration of the composition, and the composition may be administered in the form of food or feed additive, such as dietary supplement and/or component in solid form and/or in the form of a beverage. Further embodiments may be in the form of suspensions or solutions; such as the beverages further described below. Suitable forms may also be aerosols, globules, suppositories, capsules or tablets, chewable or soluble, e.g. effervescent tablets, as well as powders and other dry forms known to those skilled in the art, such as granules, for example microgranules.

The systemic administration may be parenteral, rectal, intravaginal, inhalatory and oral, in the form of additives to feed or food, as disclosed herein above. Vehicles for parenteral administration include sodium chloride solution, Ringer's solution with dextrose, dextrose and sodium chloride solution, Ringer's solution with lactates or plant oils.

The therapeutically active component may subsequently be mixed with pharmaceutically acceptable excipients compatible with the active component. Suitable excipients are, for example, water, saline, dextrose, glycerol, ethanol, or the like, and combinations thereof. In addition, if desired, the composition may contain trace amounts of auxiliary substances, such as lubricating or emulsifying agents, pH modulating agents, buffering agents, which enhance the effectiveness of the active component.

Various forms of the dermaceutic may be provided, such as solid, liquid, lyophilized, or dried otherwise. They may include diluents as for example various buffers (e.g., Tris-HCl, acetate, phosphate buffers) having various pH ranges and ionic strength, additives, such as albumin, gelatin, detergents (e.g. Tween 20, Tween 80, Pluronic F68, bile acid salts), solubilizing agents (e.g. glycerol, polyethyleneglycerol), antioxidants (e.g. ascorbic acid, sodium metabisulfite), preservatives (e.g. thimerosal, benzyl alcohol, parabens), bulking substances or tonicity modifiers (e.g. lactose, mannitol), polymers, such as polyethylene glycol, polymers forming complexes with metal ions, polylactic acid, polyglycolic acid, hydrogels, etc., or liposomes, nanocapsules, microemulsions, micelles, unilamellar or multilamellar vesicles, erythrocyte ghosts, spheroplasts or chitin derivatives.

The dermaceutic may be in a form adapted for oral administration, for example the form of a beverage, or its dry formulation, by any method in accordance with the invention.

The beverage contains an effective amount of the product in the form of *L. reuteri* DAN080 cultures, partially inactivated *L. reuteri* DAN080 cultures, liquid supernatants of *L. reuteri* DAN080 cultures, concentrated supernatants of *L. reuteri* DAN080 cultures, and dried supernatants of *L. reuteri* DAN080 cultures, or mixtures thereof, together with a water-soluble, nutritionally acceptable carrier, such as mineral components, vitamins, carbohydrates, fats and proteins. All such components are supplied in a dried form, if and when the beverage is provided in a dry form. The beverage supplied in the form ready for consumption additionally contains water. The final solution of the beverage may also have a controlled tonicity and acidity, e.g. as a buffered solution, in accordance with the general suggestions provided in the paragraph above.

The pH remains preferably within the range from 2-5, especially 2-4, for the prevention of bacterial and fungal growth. A sterilized beverage may also be used, with pH of approximately 6-8.

The beverage may be supplied alone or in combination with one or more therapeutically effective composition for topical local administration or with the medical device of the invention.

The use of *L. reuteri* DAN080 cultures, partially inactivated *L. reuteri* DAN080 cultures, liquid supernatants of *L. reuteri* DAN080 cultures, concentrated supernatants of *L. reuteri* DAN080 cultures, and dried supernatants of *L. reuteri* DAN080 cultures and other above-mentioned forms of the active component used in accordance with the present invention comprise use for manufacturing a composition for prevention amelioration or treatment of medical conditions of body surface.

Further aspects of the present invention cover uses where the dermaceutic is a pharmaceutical composition. This composition may include pharmaceutically acceptable carriers and/or additives, such as diluents, preservatives, solubilizing agents, emulsifiers, adjuvants and/or carriers useful in the use disclosed herein, in accordance with the invention.

Furthermore, as used herein, 'pharmaceutically acceptable carriers' are well known to skilled artisans and may cover, but are not limited to, 0.01-0.05 M phosphate buffer or 0.8% saline. In addition, such pharmaceutically acceptable carriers may be aqueous or non-aqueous solutions, suspensions and emulsions. Examples of non-aqueous solvents are propylene glycol, polyethylene glycol, plant oils, such as olive oil, and injectable organic esters, such as ethyl oleate. Aqueous carriers include water, alcoholic/aqueous solutions, emulsions or suspensions, including saline and buffered media. Parenteral vehicles include sodium chloride solution, Ringer's solution with dextrose, dextrose and sodium chloride solution, Ringer's solution with lactates or plant oils. Preservatives and other additives may also be present, such as antimicrobials, and antioxidants, chelating agents, inert gases, and the like.

The composition used in the kit according to the present invention, may optionally contain a carrier and/or a certain amount of a second or subsequent active component, exerting an effect on the condition of body surface and immunologic response of the patient organism.

Amelioration and prevention of body surface diseases is accomplished by administration of the composition according to the present invention on the basis of *L*. *reuteri* DAN080 cultures of, partially inactivated *L. reuteri* DAN080 cultures, liquid supernatants of *L. reuteri* DAN080 cultures, concentrated supernatant of *L. reuteri* DAN080 cultures, and dried supernatants of *L. reuteri* DAN080 cultures, and other above-mentioned forms of the antimicrobial agent of the invention. The therapeutically effective amount of is approximately 0.0001 - 0.2 g/kg of body weight per day.

As will be readily understood by one skilled in the art, the dermaceutic and medical devices of the invention are particularly suitable for humans, domestic animals, pets, animals involved in sports, broilers, laying hens, mice, rats, guinea pigs, rabbits, and other animals, e.g. laboratory animals, including the primates, wild animals (living in and/or outside zoos), irrespective of age.

It is envisaged in accordance with the invention to provide dermaceutic or medical device, which during the technological process, requiring participation of lactic fermentation bacteria, shows special pro-health properties, when making use of:
1) bacteria of genus *Lactobacillus -* in particular *L. reuteri* DAN080, showing the specific probiotic properties,
2) technology, that allows maintaining in the product living bacterial cultures. The fermentation products of such bacteria ensure maintaining bacterial homeostasis of alimentary tract.

Due to the specific antibacterial properties the fermentation products:
- prevent colonization by pathogenic bacteria, including *Helicobacter pylori,*
- protect against infections of the alimentary tract,
- ameliorate the infections, among others those caused by *Helicobacter pylori,*
- extend the shelf life of the product intended for consumption,
- when used for coating the protectors, protect the same against contamination while preserving the device itself.

Generally, the term "probiotics" refers to such microorganisms which have been commercially used as additives to food and feed, and also have found their use in the pharmaceutical industry. The definition of 'probioticum' comes from the middle 1970s, when selected microbes were used in the feeding of animals.

Probiotics enhance public health conditions, which enhancement is mainly the result of protection of the population against systemic diseases and infections, and also of weakening of the symptoms or reduction of effects of such systemic diseases. The studies on probiotics consist of the assessment of the scope within which the specific bacterial strains prevent the occurrence of disorders, and also of providing the explanation of their health promoting effect.

The health promoting activity of lactic acid bacteria consists mainly of prevention of colonization of the body surface by undesirable microbiota, while the products secreted and released by the bacteria to the extracellular surroundings, such as active compounds: acids, hydrogen peroxide (protons), enzymes, bacteriocins, or else bacterial degradation products: fragments of the bacterial wall, affect the growth of other microorganisms (including pathogenic). Post-fermentation products of lactic acid bacteria metabolism possess also a capability of decreasing the level of bacterial toxins and micotoxins (fungal metabolites).

The bacteria *L. reuteri* DAN080 are also characterized by the resistance to the effect of thermal stress occurring during the fermentation process. The production of lactic acid should be maintained by bacteria at a relatively constant level, irrespective of the temperature in which the fermentation develops and the product is stored.

The unique bacteria of the genus *Lactobacillus - L. reuteri* DAN080, are intended, among other things, to be marketed as a probiotic. The results of preliminary studies with the use of an animal model show that, following an oral administration of the fermentation products of these microorganisms to the infected (with *H. pylori* and other bacteria causing gastrointestinal tract infections) stomach of a mouse, the clear reduction of the infection occurs. The studies show that thermally stable fermentation products of the bacteria *L*. *reuteri* DAN080 improve the immune condition in infected mice, and at the same time, protect the stomach against further colonization. The observations supported by the *in vitro* studies allow the speculations that a decrease in the distribution of *H. pylori* as a result of the activity of fermentation products of the bacteria *L. reuteri* DAN080, may lead to a similar effect in humans endangered by the infections.

The characteristics of cellular metabolites observed in laboratory conditions enable the assumption that *L. reuteri* DAN080 may be widely used.

Now in turn, beneficial effect of an addition of vitamin D to the dermaceutic and medical devices according to the present invention should be explained. The role of vitamin D and its active metabolites in the defense responses of the organism covers several levels.

On the first level are epithelial cells which constitute a physical barrier protecting against injury and/or infection/invasion. Active hormone 1.25(OH)2 vitamin D enhances the physical barrier by stimulating genes encoding gap junction proteins, adherence genes, tight junction genes, and enhances intercellular communication (proteins: connexin 43, E-cadherin, occludin).

Secondly, vitamin D has a stimulatory effect on epithelial cells in the synthesis of antimicrobial peptides of innate immunity, including beta-defensins, cathelicidin LL-37.

Subsequently, vitamin D stimulates expression of potentially active antimicrobial peptides synthesized in macrophages/neutrophils, and increases the potential of oxygen burst in macrophages.

Besides, it enhances the neutralization of endotoxins through LL-37.

With respect to acquired immunity, vitamin D shows a suppressive effect, manifested as its capability for the inhibition of T lymphocytes proliferation. It exerts a suppressive effect on immunity dependent on the production of cytokines and immunoglobulins through activated B lymphocytes. It inhibits the activity of Th1 lymphocytes, and reduces synthesis by Th1 IF-gamma and IL-2 (stimulator of antibodies and cytokines). These lymphocytes participate in the development of disorders of the autoimmune background (e.g. type 1 diabetes, rheumatoid arthritis, autoimmune inflammation of the intestines, and multiple sclerosis).

According to the present invention vitamins D and E are used as commercially available nanoparticles. Nanoparticles of vitamins D and E are characterized by higher bioavailability. However, following an oral administration, the measurements of the levels of these vitamins in blood, performed within the standard periods after administration show a normal or only slightly elevated values. The measurement performed within a time shorter than the standard ones confirms an elevated bioavailability of vitamins D and E.

In accordance with the present invention, the dermaceutic contains as the active component the whole culture of *L. reuteri* DAN080, liquid supernatant, concentrated supernatant and dried or lyophilized supernatant obtained from the culture of *L. reuteri* DAN080, and from mixed bacterial cultures comprising *L. reuteri* DAN080, and liquid supernatants, concentrated supernatants and dried or lyophilized supernatants obtained from cultures of prokaryotic and eukaryotic recombinants and whole cultures of prokaryotic and eukaryotic recombinants, in which and/or from which genes are utilized, which genes provide specific modulatory, inhibitory, homeostatic activity against *H. pylori* and other bacteria; proteins/oligopeptides/peptides of the molecular weight of approximately: 150 and/or 141 and/or 115 and/or 95 and/or 90 and/or 86 and/or 83 and/or 77 and/or 71 and/or 63 and/or 59 and/or 56 and/or 49 and/or 46 and/or 43 and/or 39 and/or 34 and/or 32 and/or 30 and/or 22 kD or lower, that are purified/isolated from liquid/condensed/dried supernatant obtained from *L. reuteri* DAN080, and purified/isolated from whole cultures of *L. reuteri* DAN080, and from other mixed bacterial cultures comprising *L. reuteri* DAN080, and purified or isolated from cultures of prokaryotic and eukaryotic recombinants, in which and/or from genes are utilized, which genes provide specific modulatory, inhibitory, homeostatic activity against bacteria, or mixtures thereof useful in prophylaxis and treatment of medical conditions developing as a result of infections caused by bacteria, fungi and other pathogens of body surface (integuments) in vertebrates, finds its special use in protection of skin, as a body surface, against infections.

Similar to other probiotics, the probiotic potential of the bacteria *L. reuteri* DAN080 is evaluated based on the possibilities of passage through the gastrointestinal tract, production of antimicrobial compounds, degree of adherence to the epithelial mucin (e.g. intestinal epithelium), production of biogenic amines, mucin degradation, drug sensitivity pattern.

During fermentation, considerable amounts of final acidic metabolites are released by bacteria, accompanied by a decrease in pH. The products are difficult to quantify and include hydrogen peroxide and diacetyl, being the agents regulating microbiological relationships in the environment (antibiosis). Bacteriocins are important in the selection of microbiota triggering the fermentation. The strain/strains were identified and characterized morphologically and biochemically, as well as molecularly (identification), with respect to capabilities of survival in an acidic environment, in the presence of bile salts, capability for the utilization of proteins, starch, fats, for production of hydrogen peroxide, for bile salts hydrolase activity, and also for the production of substances inhibiting the growth of other bacteria undesirable in the patient's organism, and the determination of resistance to antimicrobial compounds. The evaluation shows non-infectiousness of the strain *L. reuteri* DAN080, which was tested on animals with impaired immunity.

Gram-positive bacteria encode proteins required for the incorporation into own cellular wall (D-alanine esters). This process, with the participation of teichoic acids, is important for the bacterial cell and its tolerance to acidic character of the environment, its resistance to antimicrobial peptides, its adhesion, formation of a biofilm, and degree of its virulence. The presence of D-alanine residues is important for the functioning of *L. reuteri* DAN080 cells and their survival in the gastrointestinal tract. It was found that treatment with catalase, changes of pH, and heating up to 80°C do not affect the bactericidal activity of *L. reuteri* DAN080. Even the treatment with trypsin and proteinase K did not affect this characteristic. No reduction in antimicrobial activity was observed when an increased availability of glucose (source of carbon) and peptone (source of nitrogen) in the medium has occurred. The bacteria *L. reuteri* DAN080 survived also in pH 3 and subsequently were not sensitive to the activity of cholic acid and bovine bile, while still exhibiting bile salts hydrolase activity, and ability to produce antimicrobial compounds.

*L. reuteri* (LR) - including *L. reuteri* DAN080, are microorganisms which may produce hazardous primary and secondary metabolites, including organic acids, diacetyl, CO₂ and various antibiotic-like substances, such as reuterin, reutericin, reutericyclin, cobalamin, etc.

Some strains of LR possess a capability of synthesizing and releasing bacteriocins. One of these is reutericin 6 - a bacteriocin, which shows both bactericidal and bacterioststic activity with respect to many species of bacteria, especially those Gram-positive. Lytic power was the strongest in the poorly opaque environment with a small number of live cells, in presence of beta-galactosidase (leakage from bacterial cells). This bacteriocin is not active against Gram-negative bacteria and does not occur in the strains of *L. reuteri* producing reuterin.

Reutericin 6 has a molecular weight of 2.7 kDa and comprises 67% of hydrophobic and polarly neutral amino acids, among which no lanthionine is found. The structure of the molecule is cyclic, and impossible to differentiate from gasericin A (similar molecular weight and amino acid sequence). Both bacteriocins differ with respect to bactericidal strength. Although they cause leakage of potassium ions from the cell and from liposomes, the strength of the leakage is different. Structurally, the bacteriocins are mainly in the form of alpha helises, with the difference in the number of amino acids with D and L configuration present. Reutericin 6 has two D-alanine residues among all 18 alanine residues present, while in gasericin there is only one such a residue. The number of amino acids residues, differing in their D or L configuration, determines the bactericidal activity of LR.

LR show anti-microbial activity, for which none of the known bacteriocins or reuterin or else organic acids are responsible. Reutericyclin shows a wide inhibitory spectrum of antimicrobial activity. Its activity does not inhibit the growth of Gram-negative microorganisms; however, *E. coli* mutant, with the LPS structure different from that of the non-mutant strain, is sensitive to the effect of reutericyclin. Reutericyclin acts against cells in a dose-dependent way. It does not destroy spores, but violates the conditions in which the germination of spores occurs. The addition of fatty acids to the bacterial culture medium changes the activity of reutericyclin. Reutericyclin - as a molecule, is hydrophobic, has a negative charge, and molecular weight of 3.49 kDa. Structurally, reutericyclin is a derivative of tetram acid (see: A. Höltzel, M. G. Gänzle, G. J. Nicholson, W. P. Hammes, and G. Jung, Angew. Chem. Int. Ed. 39:2766-2768,2000).

Reuterin production is enhanced in the presence of glycerol.

Reuterin is a substance of antimicrobial activity produced mainly by *L. reuteri* during the process of anaerobic fermentation in the presence of glycerol. Maximum production of this substance occurs in the static phase and the phase of logarithmic bacterial growth.

In the presence of glycerol, *L. reuteri* synthesizes β-hydroxypropanal (HPA) which is subsequently secreted into the medium. It was confirmed that in a water solution reuterin occurs as a mixture of three forms of β-hydroxypropanal: monomeric, hydrated and dimeric, which remain in balance.

This compound was first isolated, purified and identified by Talarico and Dobrogosz. To-date, a number of reuterin properties have been demonstrated, primarily it is an effective inhibitor of the growth of a wide spectrum of not only bacteria, but also of fungi and protozoa. The mechanism of reuterin activity has been investigated for over 20 years, and at present it is known that this compound may exert an effect on microorganisms in a dual manner. The substance may inhibit the activity of bacterial ribonucleotide reductase (an enzyme catalyzing the first stage of DNA synthesis) by competing with ribonucleotides for binding sites in the DNA sequence, or by reaction with unstable thiol groups of this enzyme. In addition, it was found that reuterin may enter into direct reactions with thioredoxin, a protein performing the role of a reducer of many enzymes, including ribonucleotide reductase, thus inhibiting the enzymatic activity of this protein. Reuterin is a substance soluble in water, acting within a wide pH values, resistant to lipolytic and proteolytic enzymes treatment. The optimum conditions for the growth and production of reuterin by *L. reuteri* is the temperature of 37°C and pH 4.6-5; this compound also remains stable in an environment of a considerably lower temperature and acidity of the medium.

The strains of LR also produce cobalamin (vitamin B12) in the process of co-fermentation of glycerol and glucose.

Genetics: It is possible to construct a shuttle vector (e.g. *Escherichia coli-*lactobacillus) transferred into *L. reuteri* DAN080 cells, so that the transformant obtained showed its activity, e.g. antimicrobial.

There is a possibility of cloning genes in *L*. *reuteri* DAN080, (known is the cloning of beta-galactosidase heterodimer in *L. reuteri* cells other than *L. reuteri* DAN080 cells), and it is assumed that the expression of such structural genes must be associated with the activity of proteins involved in maturation (cutting, cyclic form) and secretion outside the *L. reuteri* DAN080 cell by various transporting systems. It is also assumed that in *L*. *reuteri* DAN080 the mechanisms significant for the autoprotection of cells against such strong inhibitors as e.g. reutericin 6, are present.

It is possible to construct with *L. reuteri* DAN080 cells a ligated gene of the green fluorescence protein into the secretion vector which generates release of a chimeric protein capable of glittering (marker).

It is possible to adapt *L. reuteri* DAN080 cells to be incorporated into the Nisin-controlled gene expression (NICE) system by ligating nisA promoter (PnisA) and nisRK DNA fragments into the shuttle vector *E. coli-L. reuteri* pSTE32. In such a chimeric plasmid the expression of heterologic genes is possible with the induction of nisin.

In further aspect of the invention, as two types of polymers are used as a basic material for manufacturing the medical device of the invention: PVC and silicon.

PVC is a cheap polymer, the safety of which has been confirmed for many years. At present, a new generation of plasticizers is used, additionally increasing safety of PVC. Silicon is an expensive polymer; however, it is characterized by a very high compatibility.

According to the invention medical device made of PVC or silicon is coated with a polymer nanocoating made of polyvinyl pyrrolidone (PVP). Only in contact with water PVP forms a thick jelly solution which lubricates the polymer surface.

Polymer coating made of PVP may also be applied on medical devices of the invention, made of polyurethanes and natural latex.

PVP is widely used in pharmacology for the production of biomaterials intended for contact with blood and other body tissues, in results of its biocompatibility (lack of toxic effect, including degradation effect on blood cells - hemolysis, lack of effect on the host immune system). An advantage of nanocoating made of PVP is that this coating is resistant to activity of microorganisms, including pathogenic organisms.

According to the invention, nanocoating made of PVP is chemically bonded to a polymer of antimicrobial activity, such as chitosan salts. This is a polymer obtained from *Crustacea* shells. Chitosan salts are known for their use in medicine. They are safe, bioavailable and biodegradable.

In order to prove an unexpected synergism, the coatings made of chitosan salts and PVP were examined separately and in combination. In addition, the composition of individual coatings was enriched with other active substances increasing the spectrum of activity of the surface of the medical device of the invention, such as extracellular metabolites of *L. reuteri* DAN080 (deposit DSMZ - access number - DSM 15693 - in accordance with a Budapest Treaty on the International Recognition of the Deposit of Microorganisms for the Purposes of Patent Procedure, on 20 June 2003), and vitamin D in the form of nanoparticles and/or nanoparticles of silver.

Forming of solid polymer coating may be proven by two methods - physical and chemical, based on covalent polymer chains bonded through covalent bonds. Physical anchoring of polymer chains can be achieved by forming a nanocoating of discontinuous PU layer and application of PVP solution.

Due to London forces, the PVP chain is partially immersed in the basic polymer and partially protrudes therefrom. This nanocoating, having thickness of about 50,000 C-C bonds (10 nm), when immersed in water forms a sort of brush with excellent lubricative properties. This original technology has already been developed.

Alternatively, on the surface of the basic polymer, a desired layer is deposited by the method of forming free radicals by hydrogel absorption. Such free radicals are very active and easily 'catch' other chemical substances forming stable covalent bonds.

The application of both technologies is possible, as both of them provide the desired coatings.

Nanocoatings obtained on the polymer surface are tested to evaluate their biocompatibility, development of a biofilm and colonization by microorganisms. Their friction coefficient against pig tissue is also examined. The evaluation is performed making use of a specially constructed device, improved to meet the needs of the current invention. The optimum friction coefficient ensures painless contact of the medical device with the tissues.

The novel properties of the external nanocoatings of the medical device according to the invention were achieved by physical and/or chemical bonding of active agents.

The active agent of the external nanocoatings of the medical device of the invention is the component originating from the new DAN080 strain of lactic acid bacteria *L. reuteri* identified by the present inventor, deposited on June 20, 2003 - in accordance with a Budapest Treaty on the International Recognition of the Deposit of Microorganisms for the Purposes of Patent Procedure, in DSMZ collection - Deutsche Sammlung von Mikroorganismen und Zellkulturen GmbH, in Braunschweig, DE, access number: DSM 15693.

It has been now unexpectedly found out that extracellular metabolites of *L. reuteri* DAN080 are a desired component of the external nanocoating, or one of the layers which coat the medical device according to the invention, preferably the dressing or a blanket.

After the specified period of growth, the *L. reuteri* DAN080 culture is centrifuged, and the liquid, concentrated supernatant and dried supernatant is the product of specific capabilities and activity with respect to the regulation of bacteria growth *in vitro* and *in vivo.* After the electophoretic separation performed using a liquid supernatant, concentrated supernatant and dried supernatant collected at a specific time, specific proteins were visualized, the proteins having molecular weight within the range of 150 - 22 kD and smaller, which are responsible for homeostasis and bacteria growth regulation in the patient's body. These proteins, both in an isolated and purified form, and also in the form of a liquid supernatant, concentrated supernatant and dried supernatant, collected after the proper period of culturing the *L. reuteri* DAN080 culture, independently or in a mixture with other lactic acid bacteria isolated by and being the property of the present inventor - Danuta Kruszewska, confer new, unexpected properties to nanocoatings coated on the medical device according to the invention, which properties increase the safety and comfort for patients, while showing homeostatic and regulatory effect on bacterial growth on the surface of the medical device according to the invention.

Extracellular metabolites of the *L. reuteri* DAN080 are used in combination with other known agents of antibacterial activity, according to the present disclosure. The methods of isolation, culturing the *L. reuteri* DAN080 bacteria and collection, isolation and purification of extracellular metabolites of the *L. reuteri* DAN080 are described above - in the introductory part of the section directed to the detail description of the invention.

Apart from extracellular metabolites of the *L. reuteri* DAN080, vitamins are also used as active agents, especially vitamins D and E in the form of nanopowders, in order to enhance the patient's immune mechanisms, and also as agents reducing the growth of microorganisms and the formation of biofilm according to the present invention nanoparticles of silver, small dicarboxylic acid and chitosan salts may be used. In order to gradually release the above-mentioned active agents, known compositions for delayed release were used, for example compositions soluble in physiological fluids for coating the hydrogel. The selection of the used technical means for delayed release of the substances depends on the necessity of preserving their properties for the time adjusted to the anticipated period of use (contact with the patient's tissues) of the medical product according to the present invention, considering the diffusion coefficient and/or degree of bonding on the surface.

### Description of experiments

Preparation of chitosan salts: The raw material used for producing proper chitosan salts is technical chitin obtained from *Basinomycetes* (*Lentinus edodes,* Le 323), according to the publication No. PL 384836 - "Method for obtaining fungal chitosan' of 12 October 2009, or from the scutum of Antarctic krill *(Euphausia superba).* The residues of organic and inorganic contaminants are removed from the polymer in the processes of demineralization and deproteinization. A part of the chitin thus obtained is subjected to the process of chemical decomposition in order to reduce its polymerization degree to the desired level. This allows obtaining chitosans with similar deacetylation levels and different molecular weights. Chitosan is obtained in the process of alkaline deacetylation. Its properties are modified by changing the reaction time and temperature. Salts are obtained in the reaction of chitosan with organic acid(s) in aqueous environment. Thus obtained salt solution is lyophilized. The evaluation of the properties of raw material and products is monitored.

In order to obtain (in laboratory conditions) chitosans of various molecular weights and deacetylation levels the following procedure is applied:
a) preparation of the raw material for the production of chitosan: technical grade chitin is purified, the molecular weight of the polymer is modified in order to obtain chitin having various polymerization levels. Chitosan of the required properties is obtained through control of parameters of the deacetylation process.
b) Chitosan salts obtained in laboratory conditions are tested for their antimicrobial properties.

The biological testing of chitosan salts that facilitates quick assessment of their antimicrobial activity, allows monitoring, control and selection of the optimum parameters of their production process, especially the ranges within which the deacetylation level and molecular weight of chitosans should be modified.

The optimization of the method for obtaining salts is carried out from the aspect of the most intensive biological activity after the sterilization process, taking into consideration the destination of the medical product of the invention and the environment in which it will be used.

Adhesion of multi-drug-resistant bacterial strains to coated and non-coated surfaces of the polymers tested: The pathogenesis of many bacteria is associated mainly with the ability of these organisms to irreversibly adhere to polymer surface and to produce an extracellular glycocalyx in the course of colonization.

The percentage of adherence is defined as a rate of CFU recovered from the polymer tested to CFU of marker bacteria (multi-drug-resistant strains) in the culture fluid.

Antimicrobial activity of chitosan salts with relation to multi-drug-resistant microorganisms: Each sample is cultured together with one of the tested strains (CFU 10³). At various time points (0, 30, 60 and 120 min) after incubation at the temperature of 37°C samples are collected, vortexed and placed on plates plated with agar solidified medium. The CFU values are counted after incubation of microorganisms at the temperature of 37°C for 48 hours. The CFU counts in samples taken before incubation is used to calculate the reduction of CFU.

Studies on animals were based on the non-infectious animal model of the adult rats. Six-month-old Sprawgue-Dawley females rats were used with the weight of ± 350 g (n=90). The rats were divided into 9 groups. Into the urinary tract of rats (n=36; 3 groups) polymer rods were inserted covered with antibacterial and lubricous coatings. Impregnated rods were made of PVC, polyurethane and silicon, respectively. Another three groups of animals (n=36) served as negative controls in which the rats had only uncoated rods inserted into their urethra. The subsequent two groups (n=12) served as a positive controls in which the rods inserted were coated with nanosilver and PVP. The remaining rats (n=6) did not have any biomaterials inserted.

The above-mentioned rods were placed in rats from the peritoneal cavity to the urethra. They were inserted by piercing below the exposed urinary bladder, at the site between the urinary bladder and the urethra. After drilling through a micro hole in the urethra, the rod was fixed with its rounded tip towards the external wall of the urinary bladder. The length of the rod was made so that it did not protrude from the external urethral orifice, thus enabling study of the encrustation process and to avoid the rods being pulled out or bitten by the rats. The diameter of the rod had to be twice as small as the urethra diameter, and external tip of the rod had to be rounded.

The animals remained under veterinary supervision. After 7 to 14 days after the onset of the study the rats are sacrificed. Samples of urine, blood, tissues and the rods were taken in sterile conditions. Urine and blood samples were subjected to micobiological examinations.

In serum samples, lysozyme levels and defensins activity were measured.

The surface of the rods was analyzed for the colonization by microorganisms and the degree of incrustation by glycocalyx.

Isolated organisms adhering to the surface of the rods were identified and their biochemical activity characterized, including the determination of their sensitivity to antibiotics.

Prior to fixation, the tissues were analyzed for the settlement of microorganisms. Fixed samples of tissues examined morphologically and immunochemically and the presence of defensins was determined.

Characteristics of microorganisms isolated from animal tissues: It was found out that the microorganisms were identical as the strains tested (antibiogram, integrons profile, urease activity - in consent). As bacteremia/bacterinuria related with the polymer of which the rod was made, and its layer in contact with the site of activity was considered the state when the microorganism was characterized by antibiogram, integron profile, and urease activity, identical as those of the microorganism isolated from the tip or other segment of the catheter, from urine and blood of the animals in the study.

### Quantitative determinations

Bacteria isolated from blood, urine, tissues and polymer rods were counted by classical methods: Serial dilutions of the animal fluids or homogenized tissue (urethral bioptates) were plated on the appropriate media and cultured at the temperature of 37°C for 24 hours, in aerobic and anaerobic conditions. The number of bacteria was counted and calculated for 1 ml of blood/urine or 1 g of tissue as the mean value obtained from the 3 tests performed for a bioptate taken from a single animal. The obtained segments of polymer rods were cultured and counted by means of a quantitative technique. The samples were incubated on a solid medium (5% sheep blood agar) or on another growth medium, and the colonies cultured were counted after 24 h incubation at 37°C.

Bacterial identification system: Identification of bacteria was performed on the basis of based on biochemical activity characteristics of isolates, usually using detection systems API (bioMerieux, France), RT PCR.

Virulence characteristics of isolates: Quantitative analysis of activity urease of ureolytical bacteria *in vitro.*

Quantitative analysis of the bacteria examined was performed at various pH. The rate of urea conversion to ammonia was measured according to manufacturer recommendations (Wako Chemical). The urease activity was then expressed as µmol urea hydrolyzed after 1 min, 1 mg of protein. A standard curve was obtained with NH₄Cl within the range 0.1 - 20 mg N-NH₄⁺/L.

### a) Antimicrobial susceptibility profile as a marker for detecting similar strains

The minimum inhibitory concentration (MIC) was determined. Bacteria were tested using the disc diffusion method on Mueller-Hinton agar, according to CSLI guidelines. In both assays, *E. coli* ATTCC 25922, *P. aeruginosa* ATTC 27853, *S*. *aureus* ATTC 29213, *E. faecalis* ATTC 29212 were used as the reference strains for antimicrobial susceptibility testing.

### b) Presence of integrons/transposons PCR

The phenomenon of increasing selective resistance in bacteria, resulting from ineffective treatment, is one of the biggest challenges for health care. Mobile integrons are one of the mechanisms of spreading multi-drug-resistance. Integrons are able to transfer within the bacterial genome, as well as horizontally to and from integron-positive cells. In the study, the integron pattern was determined in bacterial strains isolated from the tested samples (urine, blood, tissues, polymer rods). Resistance genes localized in the integron box were analyzed by means of PCR with specific primers.

Integron profiles are useful tools in comparing isolates which are considered as being identical.

### c) Effect of the biomaterials used on innate immunity response

In natural physiological conditions the urinary tract is partially sterile. This phenomenon is related mainly to innate immunity response, and particularly to antimicrobial substances, such as defensins, cathelicidins, lactoferrin and lysozymes. Defensins and lysozyme are the best known and the most important for urinary tract immunity. Lysozyme levels were measured in serum, urine and tissue samples using the ELISA test, and enzymatic activity measured using the turbidimetric method. Selected defensins were measured in homogenized tissues using a sandwich ELISA test. Test RT-PCR is performed to analyze the expression of defensins in the cells of rat populations.

### d) Measurement of cytotoxicity of the biomaterials used

It is of the utmost importance that the material from which the inserted catheters [medical devices] are manufactured is biocompatible, therefore, different types of surfaces and surface coatings were examined.

A number of tests for compatibility are used which allow to determine whether a structure and/or a particular coating activates the innate immune system, if the tested materials induce necrosis and/or apoptosis, if the material is cytotoxic, and if it interferes with cell proliferation. The tests are performed both *in vivo* and *in vitro.* The results of the above-mentioned tests constitute a basis for the selection of a proper material for the nanocoating, which material, as such, satisfies the condition of being nontoxic, inducing minimum cell death, causing no or only limited inflammation, and evoking no overt inflammation. Throughout the investigations different materials for production of the medical devices were tested, alone or in combination with the medical device.

Several tests were applied to assess the performance of the biomaterials:
1) in conformity with established (external) standards, based on the guidelines laid out in FDA Modified Matrix (Blue Book Memorandum # G95-1, Attachement A);
2) standardized testing defined by the owner of the present invention, and
3) scientific tests aiming to extend knowledge concerning the response of the organism to the nanostructures under development.

In order to monitor the toxic effect of materials leaking from the nanocoating (urethral catheters coated in biomaterial) two rabbits are tested for each of the biomaterials used in uncoated and coated medical product of the invention for 1, 4 and 12 weeks. In brief, 4 strips of nanostructure coated or uncoated material used are inserted, respectively, into the left and right paravertebral muscles using a trocar. The rabbits are monitored for toxic response, and macroscopic evaluation of the implant site carried out periodically during the experiments.

At the end of the experiment, the animals are sacrificed, macroscopic evaluation of the implant site is performed, and a photographic record is taken for subsequent evaluation. Samples of blood and muscles are collected from the site of material implantation, and frozen or fixed in 4% paraformaldehyde for histopatologic examination. Muscle tissue is processed and embedded in paraffin, and sections are prepared and stained, and tissue sections evaluated for inflammation, necrosis, fibrosis and other indicators of a toxic interaction between muscle tissue and tested material. The effectiveness of the use of extracellular metabolites of the bacteria *L. reuteri* DAN080 as a basic active component of the nanocoating is justified by the following findings.

The experiments were performed on the effect of live *L. reuteri* DAN080 strain cultures, heat inactivated *L. reuteri* DAN080, and chitosan alpha-ketoglutarate on the immune system of laboratory animals.

Forty eight 2-month-old Sprague Dawley female rats with a weight of 140-275 g, were fed with feed adequate for the age of the animals, and watered *ad libitum.* Three days prior to experiment, all animals had catheters inserted in the jugular vein. The study was started by taking blood samples from the rats. Subsequently, the animals were administered intragastrically, by means of a gastric tube, 0.5 ml of the following preparations: suspension of the bacteria *L. reuterii* DAN080 - live and dead cells, chitosan AKG suspension, saline. Hundred twenty min. after the first blood taking the second blood sample was taken from the animals. On the second day, the rats received the same preparations once daily for 7 subsequent days. Twelve rats received live bacteria at a dose of 10⁶ cells suspended in physiological saline. The following 12 animals also received for 8 days, 10⁶ each of thermally killed cells *L. reuterii* DAN080. To the next 12 rats chitosan AKG suspension was administered, and the fourth group of animals (n=12) were administered intragastrically for 8 days, at each time 0.5 ml physiological saline. On day 8 after the final dose of the preparations administered intragastrically, blood was taken from the animals from the jugular vein. For the second time on the same day, blood was taken from all rats 120 min. after the first blood taking.

Determinations of the lysozyme activity in the blood were performed in the presence of a suspension of *Micrococcus lysodeikticus* cells of specified density, based on the absorbance value, and comparing this value with the absorbance curve plotted from a number of standard dilutions of crystal lysozyme (Sigma-Aldrich) in PBS and suspension of *M. lysodeikticus* cells of specified density. After 15, 30, 45, 60 min., incubation absorbance was measured at the wave length of 540 nm.

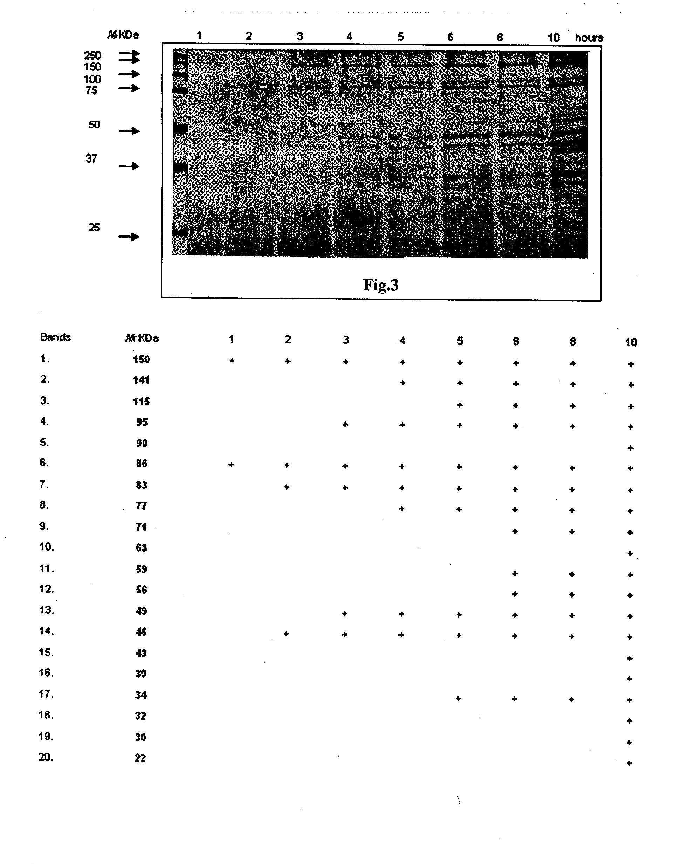

The results obtained concerning the activity of lysozyme (U/L) in the blood of rats following the intragastric administration of the tested substances, presented in Fig. 3, confirmed the stimulation of the rat immune system by live and thermally killed *L. reuteri* DAN080, and by chitosan alpha-ketoglutarate AKG.

Lysozyme is a hydrolytic enzyme released by certain phagocytes, such as macrophages and multinuclear leukocytes, plays a significant role in the control of pathogenic microorganisms. Lysozyme is also produced by Paneth cells located in the lining of the intestines. Lysozyme is especially active against Gram-positive microorganisms. Phagocytic activity of cells involves degradation, with the participation of lysozyme, of the cellular walls of bacteria, more precisely - a cleavage of glycoside bonds in peptidoglycans. An elevated lysozyme activity induced by the introduction of metabolites of *L. reuteri* DAN080 bacteria stimulated the phagocytes activation or antigen presentation to phagocytes. In this way, the function of the immune system was enhanced, mainly non-specifically. This confirms that both the live and dead cells of *L. reuteri* DAN080 show the ability to act against many pathogens. The cells as such are not recognized as dangerous by the immune system of the organism. Antimicrobial activity of the bacteria *L. reuteri* DAN080, their extracellular metabolites and chitosan alpha-ketoglutarate, is additionally enhanced, because neither live *L. reuteri* DAN080 nor their metabolites or chitosan alpha-ketoglutarate are sensitive to the activity of lysozyme, and are not hydrolyzed in contact with lysozyme.

The experiments mentioned below confirm the positive effect of the addition of extracellular metabolites of *L. reuteri* DAN080.

Behavioral tests were performed based on an open field test for the assessment of anti-anxiety effect, for the analysis of locomotive and exploratory activity under the influence of killed and live *L. reuterii* DAN080 cells on laboratory rats.

It was possible to determine the effect of those preparations on the general profile of the behavior of the animals.

Three groups of animals were administered heat treated and live *L. reuterii* DAN080 cells for three months at a dose of 10⁶ and saline at a volume of 1 ml intragastrically, using a tube. Starting from the second month of the experiment, the dose was doubled, and divided into the administration of the preparation in the morning and in the evening. Behavioral tests were performed 3 times at monthly intervals.
1. Open field test was performed 3 times, in the first, second and the third month of the experiment. The test was performed in a plastic box of the size 100 cm x 100 cm x 40 cm (height of the wall). The square floor of the box was divided by lines into 25 equal squares. The testing was performed in conditions of a quiet and bright room. Individual behaviors of the rats were observed. Each rat in the experiment was taken out of its cage and placed in the centre of the box floor.
   a. The number of squares which the rat passed during 3 min. of observation was registered.
   b. In the same box and under the same conditions an experiment was performed on the rats consisting of a 3 min. observation of the number of withdrawals of the animal's body.
   c. In the same box and under the same conditions an experiment was conducted on the rats consisting of a 3 min. observation of the number of occurrences of the snout washing and cleaning the fur.

Horizontal activity of rats was measured by the number of traversed squares. The young rats, after one month of administration of the tested preparations, showed high locomotive activity. The decrease in this horizontal activity was observed between the second and third month of the study. The least mobile, compared to the control animals, were the rats which were administered live *L. reuteri* DAN080 bacteria, followed by those receiving heat treated *L. reuteri* DAN080 (**p< 0.5, Student's t-test, *p< 0.5, *t*-test).

During the experiment, all animals with the lapse of time and - most probably, with ageing showed a tendency towards a progressive decrease in horizontal and vertical activity.

Vertical activity is measured by the number of occurrences of the animal body withdrawals.

During the first month of the administration of the preparations the young rats were mobile, and those administered live *L. reuteri* DAN080 cells for at least 3 months showed a statistically significant difference in activity, compared to the control group (*p< 0.5, *t-*test). Also, compared to the control group, a statistically significant decrease in vertical activity was noted in the rats which for 2 months had received dead *L. reuteri* DAN080 cells - (*p< 0.5, *t*-test).

In the group of animals which for 2 or 3 months received live and heat treated cells, statistically significant differences were observed (*p< 0.5, *t*-test) in the number of occurrences of the snout washing and fur cleaning, compared to the same activity performed by the control group of animals receiving exclusively saline.

In the control group, a tendency was noted towards an increase in the number of occurrences of the snout washing and fur cleaning (observation from the 1^{st} through 3^{rd} month of saline administration).

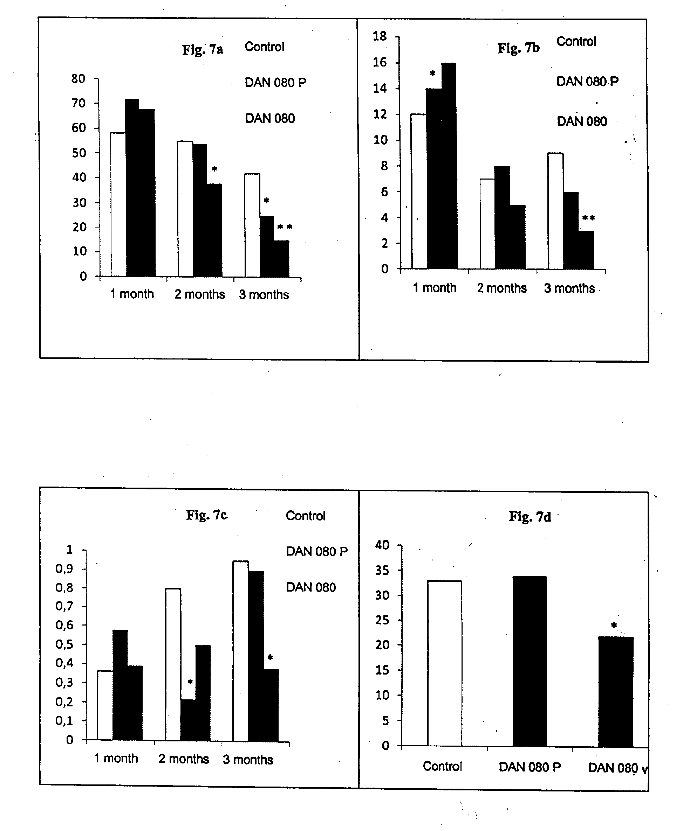

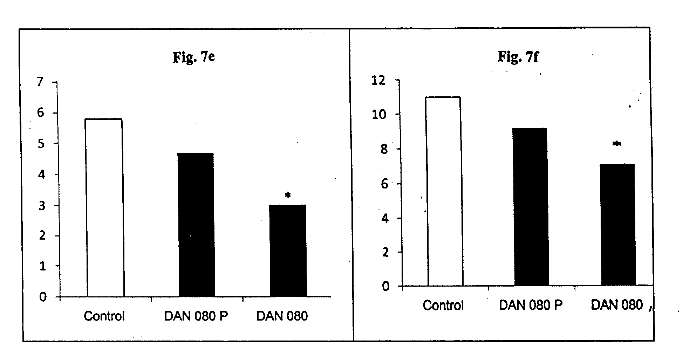

These data indicate that during the period between the 2^{nd} and 3^{rd} month of administration of the *L. reuteri* DAN080, the treatment exerted a calming effect on the rats. The results obtained are illustrated by Figures 7 a-c, wherein the number of traversed squares (Fig. 7a), the number of rat body withdrawals (Fig. 7b), and number of occurrences of the snout washing and fur cleaning (Fig. 7c) are shown.
2. Open field test - social behavior. The experiment was carried out during the 3^{rd} month of the experiment, in the same box and under the same conditions. The only difference was that 2 rats coming from 2 different cages were placed in the box. The animals received intragastrically the same preparations, according to the above-mentioned schedule of division into groups. The behavior of each pair was observed for 7 min.
   a. In the same box and under the same conditions an experiment was conducted with a pair of rats consisting of a 7-min. observation of the number of animals' body withdrawals.
   b. In the same box and under the same conditions an experiment was carried out with a pair of rats consisting of a 7-min. observation of the number of occurrences of the snout washing and fur cleaning.
   c. In the same box and under the same conditions an experiment was conducted with a pair of rats consisting of a 7-min. observation of the number of occurrences of mutual sniffing.

The number of occurrences of body withdrawals, compared to the control group, showed a statistically significant decrease in the activity of animals receiving live *L*. *reuteri* DAN080 cells (*p< 0.5, *t*-test). The statistically significant decrease in the number of the snout washing and fur cleaning, and sniffing noted in the group of rats receiving live *L. reuteri* DAN080 (**p< 0.5, Student *t*-test) indicates both a lack of stressful effect and anxiety evoking in the animals after the administration of the test preparations.

At the same time, in all the experiments conducted, no statistically significant differences were noted in the frequency of defecations and urinations by the rats. This indicates a decrease in the rats' anxiety of their new surroundings and/or new conditions.

Figures 7d-7f present the results of behavioral tests conducted on rats receiving live and heat treated *L. reuteri* DAN080 at a dose of at least 10⁶ cells/ml and physiological salt at a volume of 1 ml daily. In the open field test, the social behavior of the animals was tested by examining the number of rat body withdrawals (Fig. 7d), number of occurrences of the snout washing and fur cleaning (Fig. 7e), and number of mutual sniffings (Fig. 7f).

The experiment also confirmed a stimulatory effect of the above-tested factors in contact with epithelial cells of the mucous membranes of the body cavities other than the alimentary tract, which required catheterization.

Fig. 3 illustrates the results of electrophoresis of supernatants of the *L. reuteri* DAN080 cultures.

In the examples presented below for illustrative purposes only the test involving *H*. *pylori* were reported. The results obtain are indicative for those skilled in the art how the present invention may be evaluated and used.

### EXAMPLES

### Example 1. Effect of fermentation products of L. reuteri DAN080 and other lactic fermentation bacteria on H. pylori colonization in the mouse stomach.

48 mice (BALB/cA) divided into 4 groups of 12 mice each were involved in the study.

The first group of mice were administered daily, by a gastric tube, for 35 days, a preparation 1 (definition 1) - 0.5 ml of a mixture of neutral supernatant obtained from 10-hour culture of *L. reuteri* DAN080 cells in stationary phase, and other lactic acid bacteria, having an anti- *H*. pylori activity in combination with calcium alpha-ketoglutarate (30 mM) or chitosan alpha-ketoglutarate, or others, or other alpha-ketoacids salts administered in a liquid form, or contained in bakery products or in crisps. Starting from the 11^{th} day of the experiment, the same mice were administered twice a week for the subsequent 2 weeks, 1 hour after the first treatment a portion of 0.2 ml of fresh microscopically monitored sub-culture of *H. pylori* cells (10⁸ cells/ml) suspended in BHI.

The second group of mice was administered daily via gastric tube, for 35 days preparation 2 (limited definition 2) - 0.2 ml (10⁸ cells/ml) of *L*. *reuteri* DAN080 and other lactic acid bacteria reported in Tables 1 - 4 cells suspended in MRSB, or administered with bakery products or crisps, exhibiting anti-*H*. *pylori* activity in combination with calcium alpha-ketoglutarate (30 mM) or chitosan alpha-ketoglutarate, or others, or salts of other alpha-ketoacids, and subsequently, starting from the 11^{th} day of the experiment the mice were infected with *H. pylori* according to the infection scheme, as described for the first group.

The third group of mice was administered daily, intragastrically by a gastric tube, for 35 days preparation 3 (definition 3) - cells of *L. reuteri* DAN080 and other lactic acid bacteria, being the property of the inventor (10⁸ cells/ml) suspended in 0.5 ml MRSB, or administered with bakery products or crisps, having an anti- *H. pylori* activity in combination with a mixture of a neutral supernatant obtained from a 10-hour culture of *L*. *reuteri* DAN080 in stationary phase, and other lactic acid bacteria constituting the property of the inventor, and calcium alpha-ketoglutarate or chitosan alpha-ketoglutarate, or salts of other alpha-ketoacids.

The fourth group (positive control), twice a week for two weeks was fed by gastric tube with 0.2 ml of fresh microscopically monitored sub-culture of *H. pylori* cells (10⁸ cells/ml) suspended in BHI.

The results are shown below.

On the 36^{th} day, all mice were sacrificed and their stomachs were examined for the presence of *H. pylori* in the mucosa - Table 1.

**Table 1. Presence of H. pylori in gastric mucosa of mice from experimental groups I - IV.**

| Section of gastrointestinal tract | Colonization with *H. pylori* after previous treatment as in: | | | |
|---|---|---|---|---|
| | Group I | Group II | Group III | Group IV |
| Stomach | - | - | - | + |

### Example 2. Effect of fermentation products of L. reuteri DAN080 and other lactic fermentation bacteria on the colonization by ureolytic microbiota, in wild animals.

A diet of a group of wild animals from a zoo (n=10), without clinical symptoms indicating the disruption of the continuity of the gastrointestinal tract epithelium, constantly exposed to stress due to a rapid and permanent change of nutritional and environmental conditions, and therefore exposed to infections with ureolytic bacteria, was supplemented for 60 days, with the preparation 4 (definition 4) - being a mixture of neutral supernatant of a 10-hour culture of the cells of *L. reuteri* DAN080 in a stationary phase and other lactic acid bacteria mentioned in Tables 1- 4, or contained in bakery products or crisps, exhibiting an anti-ureolytic bacteria activity in combination with the cells of *L*. *reuteri* DAN080 and other lactic acid bacteria constituting the property of the present inventor, exhibiting the activity against ureolytic bacteria, in combination with calcium alpha-ketoglutarate (30mM) or chitosan alpha-ketoglutarate, or others, or salts of other alpha-ketoacids, or in combination with bakery products, crisps.

After the introduction of such additives to the diet/feed, and for a further 30 days of observations, the animals maintained good health and general well-being, with no fever, diarrhoea, or other symptoms of infection occurring.

### Example 3. Effect of fermentation products of L. reuteri DAN080 and other lactic acid bacteria on the colonization of the skin of the back and face of young volunteers, aged 13-17 (n=12), with the diagnosis of Acne vulgaris, by Propionibacterium acnes.

In the first group, the volunteers (n=4) were administered twice a day, for 30 days, a preparation (limited definition 5) made of a mixture of neutral supernatant obtained from a 10-hour culture of *L. reuteri* DAN080 cells in a stationary phase, and supernatants from the cultures of other lactic acid bacteria constituting the property of the present inventor, exhibiting anti-ureolytic bacteria activity with calcium alpha-ketoglutarate or sodium alpha-ketoglutarate or chitosan alpha-ketoglutarate, or others, or with salts of other alpha-ketoacids administered in the form of an ointment or moist compress.

The second group of volunteers (n=4) received twice a day, for 30 days, a preparation (limited definition 6) made of the cells of *L. reuteri* DAN080 and other lactic acid bacteria, mentioned in Tables 1 - 4, exhibiting anti-ureolytic bacteria activity in combination with calcium alpha-ketoglutarate or sodium alpha-ketoglutarate or chitosan alpha-ketoglutarate, or others, or with salts of other alpha-ketoacids in the form of an ointment or moist compress.

The third group of volunteers (n=4) was administered twice a day, for 30 days a preparation (narrowed definition 7) of cells of *L. reuteri* DAN080 and other lactic acid bacteria constituting the property of the present inventor, exhibiting anti-ureolytic bacteria activity in combination with the mixture of neutral supernatant obtained from a 10-hour culture of *L. reuteri* DAN080 in a stationary phase, and other supernatants from cultures of lactic acid bacteria constituting the property of the present inventor, having anti-ureolytic bacteria activity, and calcium alpha-ketoglutarate or sodium alpha-ketoglutarate or chitosan alpha-ketoglutarate, or others, or with salts of other alpha-ketoacids - administered in the form of an ointment or moist compress.

In the course of the study, in all the volunteers, healing was observed of the infected sites where *Acne vulgaris* occurred. No new foci of acne developed in any of the volunteers. During the 15-day observation, after termination of the administration of the preparations, no reinfection was noted.

**Table 2. Presence of P. acnes in the skin of the back and face of the volunteers infected (Groups: I - III).**

| Skin | Colonization by *P*. *acnes* after previous treatment | | |
|---|---|---|---|
| | Group I | Group II | Group III |
| Back | - | - | - |
| Face | - | - | - |

It was unexpectedly found out that the preparation is effective against chronic, porous fissure-like infections with ureolytic bacteria, including infections of the feet, armpits and groin, and epidermis products, such as nails, hair, hooves and horns. The preparation protects and reduces infections in the form of abscesses and boils, as well as sycosis, exfoliative dermatitis of infants, erysipelas, impetigo contagiosa, ecthyma, folliculitis, *Acne vulgaris,* difficult to treat erythrasma caused by *Propionibacterium minutisimum,* infections of surgical and burn wounds, and bed sores. Due to its activity reducing the colonization of the skin surface and skin products, the unpleasant odour caused by changes in the pH of the skin, and the presence on its surface of odorous extracellular metabolites of ureolytic, bacteria become eliminated.

Lactic acid bacteria constituting the property of the present inventor are easily cultured on liquid or solid media MRS (de Man Rogosa Sharpe) for 24 hours at the temperature of 37°C, in microaerophilic conditions. The above-mentioned strains show characteristics which may be considered as conducive to the colonization of the gastriointestinal tract. They possess a capability of binding matrix proteins, especially collagen and fibronectin, which favours the adhesion to the epithelium of the intestine. These bacteria release proteases causing the decomposition of milk proteins, and fermentation of saccharides contained in milk, which facilitates access of the microorganisms to the nutritional substrate. Besides, for some of them inulin may be the source of carbon. Therefore, while fermenting this indigestible fructan, irrespective of other biochemical activities biased against ureolytic bacteria, the bacteria participates in the regulation of the local intestinal microbiota. All strains survive for one hour in a medium containing 20% of bovine bile, in acidic conditions, in pH of 2.5 for 2 hours, which indicates that after oral administration they may pass intact via the stomach and small intestine to the large intestine. They do not develop resistance to antibiotics and chemotherapeutics to the degree that they would be disqualified as microorganisms potentially settling the gastrointestinal tract in humans and animals.

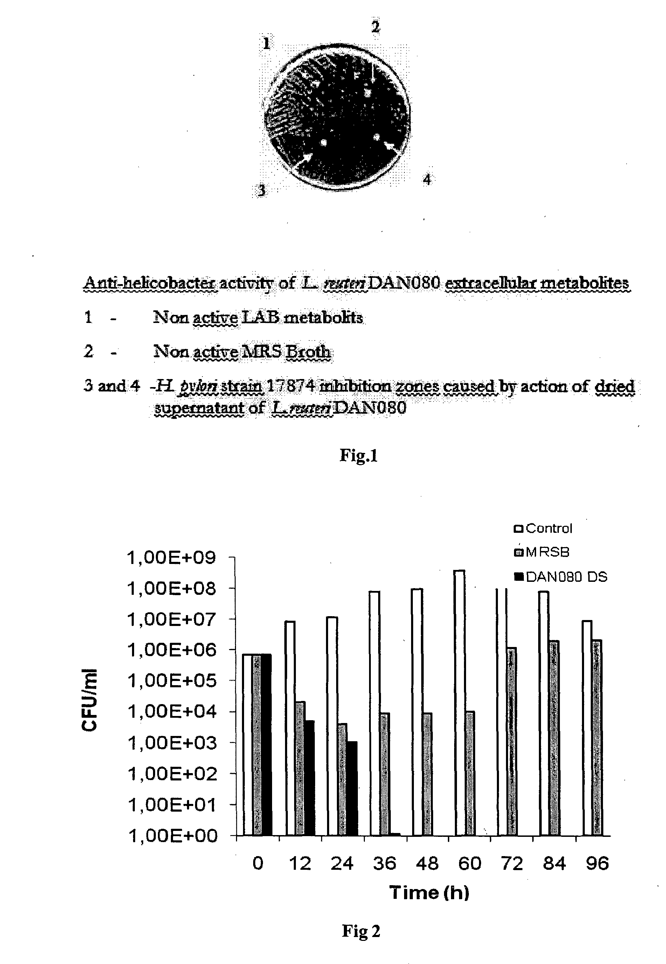

It was confirmed that the following bacteria show activity against ureolytic pathogens of the gastrointestinal tract, urinary tract, body surface, and respiratory system:
- bacteria *L. reuteri* DAN080 with bactericidal effect on *H. pylori* and other pathogens of the gastrointestinal tract by their extracellular metabolism products (Fig. 1).
- other lactic acid bacteria constituting the property of the present inventor, which during their growth maintain the capability of releasing alpha-ketoglutarate into the environment as one of their metabolites. Alpha-ketoglutarate in turn, acting locally, in the appropriate concentrations (30 mM), hydrolyses urea present in the environment, thus interfering with the process of colonization by other bacteria - ureolytic pathogens, the growth of which is dependent of the pH of the microenvironment, and is impossible in, e.g. acid pH of the stomach.

Within the region of the gastric mucosa this phenomenon covers not only such bacteria as *H. pylori,* but also *Proteus mirabilis, Citrobacter freundii, Klebsiella pneumoniae, Enterobacter cloacae, Staphylococcus aureus, Staphylococcus capitis urealiticum* (see: Osaki T et al. Urease-positive bacteria in the stomach induce a false-positive reaction in a urea breath test for diagnosis of Helicobacter pylori infection. J Med Microbiol. 2008; 57:814-9; Brandi G et al. Urease-positive bacteria other than Helicobacter pylori in human gastric juice and mucosa. Am J Gastroenterol. 2006;101(8):1756-61), which use their own urease for urea decomposition, thus providing themselves with habitation microniches.

### Example 4. Activity tests in vivo

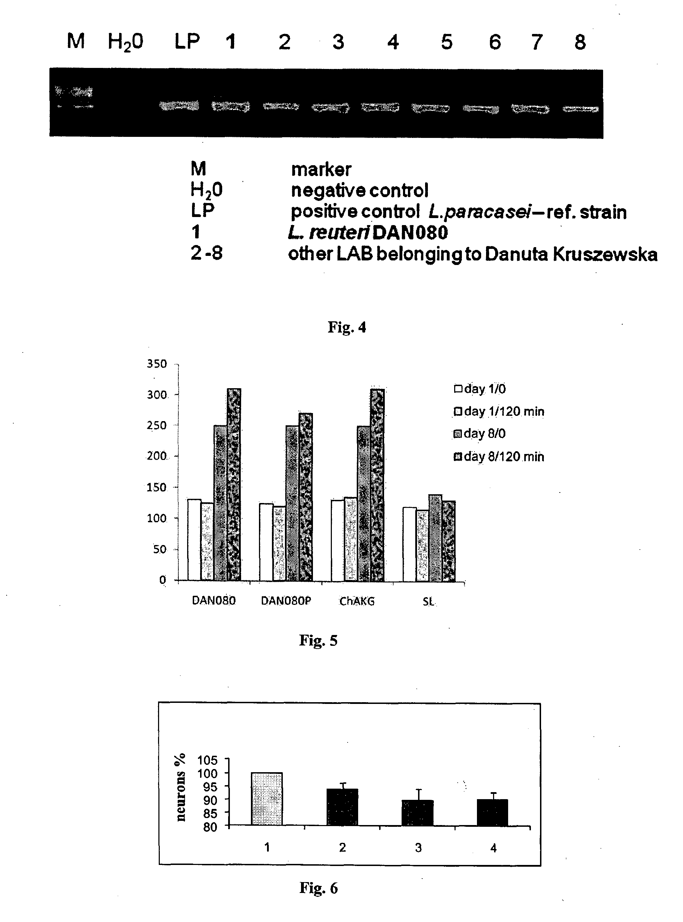

a) Activity of lysozyme: Forty eight 2-month-old Sprague Dawley female rats with a weight of 140-275 g, were fed with feed adequate for the age of the animals, and watered *ad libitum.* Three days prior to experiment, all animals had catheters inserted in the jugular vein. The study was started by taking blood samples from the rats. Subsequently, the animals were administered intragastrically, by means of a gastric tube, 0.5 ml of the following preparations: suspension of the bacteria *L. reuteri* DAN080 - live and dead cells, chitosan AKG suspension, saline. Hundred twenty min. after the first blood taking the second blood sample was taken from the animals. On the second day, the rats received the same preparations once daily for 7 subsequent days. Twelve rats received live bacteria at a dose of 10⁶ cells suspended in physiological saline. The following 12 animals also received for 8 days, 10⁶ each of thermally killed cells *L. reuteri* DAN080. To the next 12 rats chitosan AKG suspension was administered, and the fourth group of animals (n=12) were administered intragastrically for 8 days, at each time 0.5 ml physiological saline. On day 8 after the final dose of the preparations administered intragastrically, blood was taken from the animals from the jugular vein. For the second time on the same day, blood was taken from all rats 120 min. after the first blood taking.
   Determinations of the lysozyme activity in the blood were performed in the presence of a suspension of *Micrococcus lysodeikticus* cells of specified density, based on the absorbance value, and comparing this value with the absorbance curve plotted from a number of standard dilutions of crystal lysozyme (Sigma-Aldrich) in PBS and suspension of *M. lysodeikticus* cells of specified density. After 15, 30, 45, 60 min., incubation absorbance was measured at the wave length of 540 nm.
   Results: A stimulation of rat immune system was observed by live and thermally killed bacteria *L. reuteri* DAN080 and by chitosan AKG. Fig. 5 presents the results.
b) The effect of *L. reuteri* DAN080 metabolites on neurons of the enteric nervous system in swine: Neurons of the enteric nervous system were isolated from the middle section of the small intestine in 3-6 -week old piglets (n=5) with a body weight of 15 kg. The tissues were treated with tripsin, type 2 collagenase and protease in order to obtain neuron cultures. The neuron cultures were plated in Neurobasal A medium for neurons, enriched with an additive of fetal bovine serum, or in the presence of neutralized metabolites of *L. reuteri* DAN080, and twice or four-times concentrated samples of metabolites. The cultures were maintained in an atmosphere of 5% CO₂ at 37°C for 6 days.
   Results: After 6 days of incubation, it was observed in the control samples (neurons cultured on medium) that 53.7 ± 2.7% of cells survived. Considering this fact, the index of neurons was determined which survived in the presence of *L. reuteri* DAN080 metabolites, with relation to the neurons incubated exclusively on the enriched Neurobasal A medium. The cells of *L. reuteri* DAN080 isolated from mouse stomach, through their metabolites, do not statistically significantly decrease the survival rate of neurons isolated from the nervous system in piglets. The cells of *L. reuteri* DAN80 do not cause the degeneration of the nervation (structure) of the gastrointestinal tract. Fig. 6 presents the results.

### Example 5.

The commercially available catheters made of PVC (e.g. Galmed PL), not covered with any protective coating were processed using the methods of surface nanoengineering.

Nanocoating made of PVP was deposited after forming previously an intermediate layer on the basis of chitosan [salts], by a known method of submerging in the solution and air drying at room temperature, and optionally cross-linking by a short-term exposure to the UV radiation and/or to ultrasounds. According to the intended use of the catheter, the thickness of the intermediate layer was regulated by repeating the procedure of applying the first intermediate coating.

The intermediate coating on the basis of chitosan salts was enriched with active substances selected from a group covering thermally inactivated cultures of *L. reuteri* DAN080, chitosan alpha-ketoglutarate, small dicarboxylic acid, triclosan, silver nanoparticles, and vitamins D and E in the form of nanopowder coated with a protective coating.

Surface nanoengineering allows the manufacturing of an intermediate coating of the thickness of approximately 50,000 C-C bonds (10 nm).

The PVP polymer nanocoating of the indicated thickness, in the environment of physiological pH, is totally dissolved during usage. Gradual dissolving of the PVP layer reveals gradually the intermediate coating, wherefrom gradually active substances are released by diffusion. A single intermediate coating maintains its durability for the period up to one week, preventing the formation of biofilm, development of irritations and inflammatory states.

After 7 days of the catheter presence in the bodies of patients subjected to the urinary tract catheterization, in healthy volunteers no undesirable responses and reactions were noted.

Present studies concerning the catheters coated with hydrogel nanocoating show, for the first time, promising results with respect to the possibility of reduction of the frequency of CAUTI occurrence.

Even when a catheter is manufactured from non-invasive materials, these materials are recognized as foreign bodies by the cells of the immune system. The catheter according to the invention with currently disclosed external nanocoatings, in contact with the epithelial cells of a patient, does not exert any cytotoxic effect on the epithelium. Nanoparticles used in the study are not recognized as dangerous by the host cells. When evaluating a potential biological effect of the new material, consisting of exposing the antibacterial nanocoating to the liver tissues, the analysis showed a lack of hepatocytes reaction to the insertion of the catheter into the catchment area of the portal vein. A considerable improvement of the catheter was proposed with respect to the materials used. Due to the natural antibacterial coating, the new generation of catheters coated with hydrogel is characterized by lower contact friction, and reduces the urinary tract inflammatory processes and infections in a way similar to traditional antibiotic therapy. The catheter is cheaper and more beneficial for patients, as compared to traditional catheters.

### Example 6.

The kit according to the invention comprises a medical device of the invention and a stress-reducing agent for oral administration, in the form of live or heat inactivated *L*. *reuteri* DAN080 cultures, at a dose of 10⁶ cells, for everyday administration for at least the period of contact of the product with the patient's tissues. Depending on the patient status, the physician in charge may order administration of a stress-reducing agent also during the period preceding the contact with the medical device.

### Example 7.

Topical application of *L. reuteri* DAN080 culture has been tested for the prevention of superficial skin and bum wound infections. Studies involving the use of *L. reuteri* DAN080 culture (immobilized from calcium chitosan or calcium alginate films) investigated the antibacterial activity of these films in a burn wound model in rats. A multiresistant clinical isolate, ureasepositive *Pseudomonas aeruginosa,* served as the indicator strain. Films incorporating *L. reuteri* DAN080 culture (equilibrated to cell concentrations of 10⁸ CFU/mL) caused a reduction of 5-6 log(10) in *P. aeruginosa* in the model bum wounds. Wound dressings containing immobilized *L. reuteri* DAN080 culture in freeze-dried calcium chitosan or alginate films remained viable for six months of storage at 4 °C. This indicates that *L. reuteri* DAN080 culture and/or its by-products express potential therapeutic activities for the local treatment of *P*. *aeruginosa* burn infections.

The bum-mouse model of (Rumbaugh KP et al. Contribution of quorum sensing to the virulence of *Pseudomonas aeruginosa* in burn wound infections. Infect Immun1999;67:5854-5862), was used in the study. Anaesthetized mice whose backs were shaved, were placed in a water bath at a temp. 90 C for 10 s to burn the neck surface. One group of mice(B group) randomly selected, obtained a PBS injection directly under the bum and the second group was infected with 100ul of 200-300 CFU of *P*. *aeruginosa* (BPs group), half of the mice from the second group was treated with *L. reuteri* DAN080 culture on days 3, 4, 5,7 and 9 after the initial infection with *L. reuteri* DAN080 culture (equilbrate100ul of 10⁵ DAN080 cells grown in MRS broth (BPs + DAN080group). On days 5,10, 15 after the initial infection, mice were sacrificed and blood samples, skin, connective tissue and muscle from the bum area were collected and processed. Histological studies showed that on day 5 edema, vascular congestion and necrotic areas containing inflammatory infiltrates developed, these infiltrates were larger in groups BPs and BPS + DAN080than in group B. On day 10 the wound repair process was significantly advanced in group B compared to the other groups. In group BPs + DAN080 the necrosis area was smaller and the inflammatory infiltrates more diffuse than in the mice from BPs group. At day 15, 62% of the mice in group BPs + DAN080 showed a clearance of bacteria, compared to 38% in group BPs.

## Claims

1. Use of *Lactobacillus reuteri* DAN080 strain with deposit number DSM 15693 as an active component for manufacturing a dermaceutic effective in treatment of skin infections in vertebrate, in particular human being, other mammal or bird.

2. Use of *Lactobacillus reuteri* DAN080 strain with deposit number DSM 15693 as an active component exerting an antimicrobial activity, for manufacturing a medical device in the form of a dressing and/or hygienic materials for use in wound care and treatment, and in personal hygiene - respectively, to avoid skin infections in vertebrate, in particular human being, other mammal or bird.

3. Use of *Lactobacillus reuteri* DAN080 strain with deposit number DSM 15693 as an active component exerting an antimicrobial activity, for manufacturing a medical device in the form of plastic protector sheet and fire-blanket intended for rescue units, including fire-brigades, specifically for patients with extensive skin bums and for victims of communication accidents with serious body surface injuries, to avoid skin infections in vertebrate, in particular human being, other mammal or bird.

4. Use according to any one of the preceding claims 1-3, wherein *Lactobacillus reuteri* DAN080 strain with deposit number DSM 15693 in form of a culture, a partially inactivated culture; liquid, concentrated and dried supernatant of *L. reuteri* DAN080 with deposit number DSM 15693 culture, is used as therapeutic and prophylactic agent, especially as an antimicrobial agent, in prophylaxis and treatment of medical conditions developing as a result of infections caused by bacteria, fungi, and other pathogens of the body surface in vertebrate, in particular human being, other mammal or bird, and/or for increasing the activity of lysozyme in an organism of vertebrate, especially human being, other mammal or bird.

5. Use according to any one of the preceding claims 1-3, wherein *Lactobacillus reuteri* DAN080 strain with deposit number DSM 15693 in form selected from the group comprising the whole *L. reuteri* DAN080 strain with deposit number DSM 15693 culture; liquid, concentrated and dried supernatant obtained from *L. reuteri* DAN080 strain with deposit number DSM 15693 culture and from mixed bacterial cultures containing *L*. *reuteri* DAN080 strain with deposit number DSM 15693; and liquid, concentrated and dried supernatants obtained from the cultures of prokaryotic and eukaryotic recombinants, and the whole cultures of prokaryotic and eukaryotic recombinants, in which and/or from which genes are utilized, which genes provide specific modulatory, inhibitory, and homeostatic activity with respect to targeted bacteria; proteins/oligopeptides/peptides of molecular weight of approximately: 150 and/or 141 and/or 115 and/or 95 and/or 90 and/or 86 and/or 83 and/or 77 and/or 71 and/or 63 and/or 59 and/or 56 and/or 49 and/or 46 and/or 43 and/or 39 and/or 34 and/or 32 and/or 30 and/or 22 kD and lower, purified/isolated from liquid, concentrated, dried supernatants, obtained from *L. reuteri* DAN080 strain with deposit number DSM 15693 cultures; and purified/isolated from the whole *L. reuteri* DAN080 strain with deposit number DSM 15693 cultures and from other mixed bacterial cultures, which include *L. reuteri* DAN080 strain with deposit number DSM 15693; and from purified or isolated cultures of prokaryotic and eukaryotic recombinants, in which and/or from which genes are utilized, which genes provide specific modulatory, inhibitory, and homeostatic activity with respect to bacteria or their mixtures, is used as an active component of antimicrobial properties, for manufacturing medicaments for prophylaxis and treatment of medical conditions developing as a result of infections caused by bacteria, fungi, and other pathogens of the body surface in vertebrate, in particular human being, other mammal or bird.

6. Use according to any one of the preceding claims 1 to 5, wherein *Lactobacillus reuteri* DAN080 strain with deposit number DSM 15693 is used as an active component of antimicrobial activity, for manufacturing a medicament - prophylactic and therapeutic, for increasing the activity of lysozyme in an organism of vertebrate, wherein the vertebrate is a human individual.

7. Use according to any one of the preceding claims 1 to 5, wherein *Lactobacillus reuteri* DAN080 strain with deposit number DSM 15693 is used as an active component of antimicrobial activity, for manufacturing a medicament - prophylactic and therapeutic, for increasing the activity of lysozyme in an organism of vertebrate, wherein the vertebrate is a domestic animal, pet, animal involved in sport, broiler, layer hen, mouse, rat, guinea pig, rabbit and other laboratory animals, including primates, independent of its age.

8. Use according to any one of the preceding claims 1 to 5, wherein *Lactobacillus reuteri* DAN080 strain with deposit number DSM 15693 is used as an active component of antimicrobial activity, for manufacturing a medicament for prophylaxis or treatment of skin infections in vertebrate, wherein the microorganism is a pathogenic bacterium or fungus.

9. The use according any one of the preceding claims 1 to 8, wherein the dermaceutic and/or the medical device comprises a composition designed for killing, inhibiting, regulating, and preventing the growth of microorganisms, comprising said active component in an effective amount necessary for obtaining the desired preventive or therapeutic result.

10. The use according to claim 9, wherein the composition is a pharmaceutical composition optionally containing other biologically active substances, such as vitamins, especially D and E, in particular in form of nanoparticles, at preventive or therapeutic doses, pharmaceutically acceptable carriers and/or other adjuvants, as well as antimicrobial chitosan salts, especially chitosan alpha-ketoglutarate, citrate and lactate.

11. The use according to claim 10, wherein the pharmaceutical composition is additionally intended for administration *per os* and is in solid form, divided into the unitary dosages containing a therapeutically effective amount of said active component of antimicrobial activity and increasing activity of lysozyme in an organism of vertebrate, in amount of 0.001 to 0.2 g/kg of body weight per day.

12. The use according to claim 11, wherein the pharmaceutical composition intended for administration *per os* is in the form of a tablet or capsule.

13. The use according to claim 10, wherein the pharmaceutical composition is optionally additionally intended for administration *per os* and is in liquid form, divided into the unitary dosages containing a therapeutically effective amount of said active component of antimicrobial activity and increasing activity of lysozyme in an organism of vertebrate, in amount of 0.001 to 0.2 g/kg of body weight per day, in particular in ampoules.

14. The use according to claim 13, wherein the pharmaceutical composition is in liquid form and is designed for a topical use, as aerosol, cataplasm or moist compress.

15. The use according to any one of the claims 1-14, wherein the therapeutically or preventively effective amount of the active component ranges from 0.001 to 0.2 g/kg of body weight per day.

16. A dermaceutic manufactured in accordance with claim 1, which is in form of ointment, cream, lotion or other semi-solid or liquid form and contains the active component in amount adjusted to administration in amount of 0,001 to 0,2 g/kg of body weight per day when applied once a day or more than once a day, in combination with suitable vehicle and adjuvants.

17. A medical device manufactured in accordance with claim 2, wherein the device is in the form of a dressing or hygienic materials for use in a wound care and personal hygiene - respectively, such as dressing, diapers, tampons, bandages, bandaids, sanitary pads, sanitary napkins with wings, panty liners, cosmetic pads, wraps for animals for night and day use or other hygienic materials, and contains *L. reuteri* DAN080 strain with deposit number DSM 15693 culture immobilized on calcium chitosan or alginate film.

18. A medical device manufactured in accordance with claim 3, wherein the device is in form of the plastic protector sheet or the fire-blanket including at least one layer saturated or coated with a nanolayer comprising *Lactobacillus reuteri* DAN080 strain with deposit number DSM 15693 culture or extracellular metabolites secreted by *Lactobacillus reuteri* DAN080 strain with deposit number DSM 15693, said metabolites having antimicrobial and anti-inflammatory activity, as well as an optional addition of vitamin D in form of nanoparticles and preferably releasing the active component in contact with the burned or injured patient's body surface in antimicrobially and antiinflamatory effective amount.

19. A medical device according to claim 18, wherein the device is made of plastic and is coated with a protective layer and wherein it has an outer nanocoating of a biocompatible polymer, optionally able to form gel with water, permanently attached to said plastic either directly or through a nanocoating of a polymer chemically bonded to the device surface material, and having antibacterial properties, wherein at least one of the nanocoatings comprises an addition of *Lactobacillus reuteri* DAN080 strain with deposit number DSM 15693 culture and/or extracellular metabolites secreted by said *Lactobacillus reuteri* DAN080 strain, having antimicrobial and anti-inflammatory activity, as well as an optional addition of vitamin D in form of nanoparticles and wherein the biocompatible polymer is polyvinyl pyrrolidone, while the nanocoating made of this polymer has a thickness of about 50,000 C-C bonds (10 nm).

20. The medical device according to claim 19, wherein the polymer having antibacterial properties is a salt of chitosan and small organic acid, preferably alpha-ketoglutaric acid and the additional active agents are selected from the group comprising chitosan alpha-ketoglutarate, chitosan citrate, chitosan lactate exhibiting antimicrobial and anti-inflammatory activity, small dicarboxylic acid, silver nanoparticles and vitamins D and E in the form of nanopowder coated with a protective coating, and the combinations thereof and said additional active agents are dispersed in the nanocoating made of biocompatible polymer and/or in the polymer having antibacterial properties.

21. A medical kit comprising the dermaceutic and/or the medical device according to any one of the preceding claims, and a stress-reducing agent for administration *per os* in the form of live or thermally inactivated *Lactobacillus reuteri* DAN080 strain with deposit number DSM 15693 cultures at a dose of 10⁶ cells, for daily administration during treatment with dermaceutic or during contact of patient's body tissues with the medical device.
